(19) Europäisches Patentamt
European Patent Office
Office européen des brevets

(11) Veröffentlichungsnummer: **0 362 695 B1**

(12) # EUROPÄISCHE PATENTSCHRIFT

(45) Veröffentlichungstag der Patentschrift: **03.04.91 Patentblatt 91/14**

(51) Int. Cl.$^5$: **C07D 487/04**, A61K 31/40

(21) Anmeldenummer: **89117957.4**

(22) Anmeldetag: **28.09.89**

(54) **Pyrrolocarbazol-Derivate, Verfahren zu deren Herstellung und deren Verwendung als Arzneimittel.**

(30) Priorität: **29.09.88 DE 3833008**

(43) Veröffentlichungstag der Anmeldung: **11.04.90 Patentblatt 90/15**

(45) Bekanntmachung des Hinweises auf die Patenterteilung: **03.04.91 Patentblatt 91/14**

(84) Benannte Vertragsstaaten: **AT BE CH DE ES FR GB GR IT LI LU NL SE**

(56) Entgegenhaltungen:
**CANADIAN JOURNAL OF CHEMISTRY, Band 60, 1982; G.W.BRYAN REED et al. "The regioselectivity of the formation of dihydro- and tetrahydrocarbazoles by the Fischer indole synthesis" Seiten 419-424**
**HETEROCYCLES, Band 27, Nr. 5, 1988; ULF PINDUR "New diels-alder reactions with vinylindoles: a regio- and stereocontrolled access to a mellated indoles and derivatives" Seiten 1253-1268**

(56) Entgegenhaltungen:
**CHEMICAL ABSTRACTS, Band 71, Nr. 5, 4. August 1969, Columbus, Ohio, USA; RASHYDIAN et al. "Isoindoline derivatives. I. Synthesis of some diamines of the isoindoline series" Seite 311, Zusammenfassung-Nr. 21972z**
**QUARTERLY REVIEWS, Band 22, 1968; J.I.G.-CADOGAN: "Reduction of nitro- and nitroso-compounds by tervalent phosphorus reagents" Seiten 222-251**

(73) Patentinhaber: **GÖDECKE AKTIENGESELLSCHAFT**
**Salzufer 16**
**W-1000 Berlin 10 (DE)**

(72) Erfinder: **Kleinschroth, Jürgen, Dr.**
**Ricarda Huch-Str. 11**
**W-7809 Denzlingen (DE)**
Erfinder: **Hartenstein, Johannes, Dr.**
**Fohrenbühl 23**
**W-7801 Stegen-Wittental (DE)**
Erfinder: **Schächtele, Christoph, Dr.**
**Spechtweg 31**
**W-7800 Freiburg (DE)**
Erfinder: **Rudolph, Claus, Dr.**
**Riedmattenstr. 11**
**W-7801 Vörstetten (DE)**
Erfinder: **Dooley, David James, Dr.**
**Bernerstr. 22**
**W-7800 Freiburg (DE)**
Erfinder: **Weinheimer, Günter, Dr.**
**Sachsenstr. 4**
**W-7809 Denzlingen (DE)**

EP 0 362 695 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue Pyrrolocarbazol-Derivate der allgemeinen Formel I

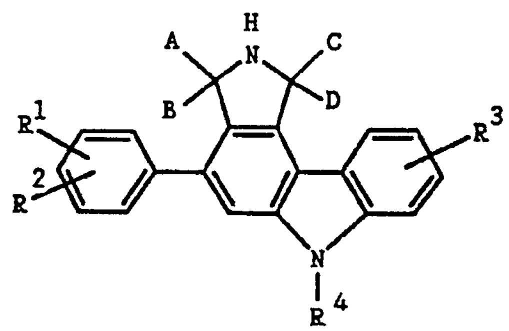

(I)

in welcher A und B oder C und D entweder gleich sind und Wasserstoff bedeuten oder zusammen ein Carbonylsauerstoffatom bilden, oder verschieden sind, wobei einer der Reste von A und B oder von C und D für Wasserstoff steht und der andere von den beiden Resten eine Hydroxygruppe bedeutet, mit der Maßgabe, daß mindestens eine der Kombinationen A und B oder C und D zusammen ein Carbonylsauerstoffatom bilden, $R^1$, $R^2$ und $R^3$ entweder gleich oder verschieden sind und jeweils für Wasserstoff, ein Halogenatom, insbesondere Fluor, Chlor oder Brom, eine Trifluormethylgruppe, eine Alkylgruppe mit 1 bis 4 C-Atomen, eine Nitrogruppe, eine Aminogruppe, unsubstituiert oder substituiert durch eine Alkylgruppe mit 1 bis 4 C-Atomen oder eine Benzylgruppe, eine Alkoxygruppe mit 1 bis 4 C-Atomen, eine Hydroxygruppe, eine Acylgruppe mit 1 bis 4 C-Atomen oder eine Aminoalkoxygruppe mit 1 bis 12 C-Atomen, unsubstituiert oder substituiert an der Alkylkette durch Alkylreste mit 1 bis 4 C-Atomen, Hydroxy oder Alkoxy mit 1 bis 4 C-Atomen und/oder substituiert am Stickstoffatom durch Alkylreste mit 1 bis 4 C-Atomen oder Benzyl oder bei der zwei Substituenten am Stickstoffatom oder ein Substituent am Stickstoffatom mit einem Substituenten der Alkylkette und zusammen mit dem Stickstoffatom einen heterozyklischen Ring mit 3 bis 6 C-Atomen bilden, der auch Sauerstoff-, Schwefel- und/oder weitere Stickstoffatome enthalten und durch Alkylreste mit 1 bis 4 C-Atomen substituiert sein kann, stehen und $R^4$ für Wasserstoff, eine geradkettige oder verzweigte Alkylgruppe mit 1 bis 6 C-Atomen, eine Cyanoalkylgruppe mit 2 bis 4 C-Atomen, einen Alkoxycarbonylalkylrest mit bis zu 7 C-Atomen oder eine Aminoalkylgruppe mit 1 bis 12 C-Atomen, unsubstituiert oder substituiert an der Alkylkette durch Alkylreste mit 1 bis 4 C-Atomen, Hydroxy oder Alkoxy mit 1 bis 4 C-Atomen und/oder substituiert am Stickstoffatom durch Alkylreste mit 1 bis 4 C-Atomen oder Benzyl oder bei der zwei Substituenten am Stickstoffatom oder ein Substituent am Stickstoffatom mit einem Substituenten der Alkylkette und zusammen mit dem Stickstoffatom einen heterozyklischen Ring mit 3 bis 6 C-Atomen bilden, der auch Sauerstoff-, Schwefel- und/oder weitere Stickstoffatome enthalten und durch Alkylreste mit 1 bis 4 C-Atomen substituiert sein kann, steht, sowie deren pharmakologisch unbedenkliche Salze, Verfahren zur Herstellung der Verbindungen I oder von Regioisomerengemischen aus zweien dieser Verbindungen I sowie Arzneimittel mit einem Gehalt an mindestens einer der Verbindungen I.

Bevorzugt sind Verbindungen der allgemeinen Formel I, in denen $R^1$, $R^2$ und $R^3$ entweder gleich oder verschieden sind und Wasserstoff, Fluor, Chlor, Brom, eine Trifluormethyl-, Hydroxy-Nitro-, Methyl-, Ethyl-, n-Propyl-, Isopropyl-, n-Butyl-, Amino-, Methoxy-, Ethoxy-, Aminoethoxy-, Aminopropoxy-, Dimethylaminoethoxy-, Dimethylaminopropoxy-, Diethylaminoethoxy-, N-Benzyl-N-methylaminoethoxy-, N-Benzyl-N-methylaminopropoxy-, Dimethylaminohydroxypropoxy-, Piperidinoethoxy-, Piperidinopropoxy-, Pyrrolidinoethoxy-, Pyrrolidinopropoxy-, Morpholinoethoxy-, Morpholinopropoxy-, Pyrrolidinylmethoxy-, Piperidinylmethoxy-, N-Methylpiperidinylmethoxy-, N-Methyl-pyrrolidinylmethoxy-, Dimethylaminomethoxypropoxy-, Formyl-, Acetyl, Propionyl- oder eine Butyrylgruppe bedeuten und $R^4$ Wasserstoff, eine Methyl-, Ethyl-, n-Propyl-, Isopropyl-, n-Butyl-, Aminoethyl-, Aminopropyl-, Dimethylaminoethyl-, Dimethylaminopropyl-, Diethylaminoethyl-, N-Benzyl-N-methylaminoethyl-, N-Benzyl-N-methylaminopropyl-, Dimethylaminohydroxypropyl-, Diethylaminohydroxypropyl-, Piperidinoethyl-, Piperidinopropyl-, Pyrrolidinoethyl-, Pyrrolidinopropyl-, Morpholinoethyl-, Morpholinopropyl-, Pyrrolidinylmethyl-, Piperidinylmethyl-, N-Methylpiperidinylmethyl-, Dimethylaminomethoxypropyl-, Diethylaminomethoxypropyl-, N-Methyl-pyrrolidinylmethyl-, Cyanomethyl-, Cyanoethyl-, Methoxycarbonylethyl-, Ethoxycarbonylethyl- oder Methoxycarbonylmethylgruppe bedeutet.

Die Herstellung der Verbindungen I erfolgt nach einem der im folgenden beschriebenen Verfahren :

A) Verbindungen der allgemeinen Formel I, bei denen sowohl A und B als auch C und D ein Carbonylsauerstoffatom bilden und $R^4$ für Wasserstoff steht (Verbindungen Ia) können gemäß Reaktionsschema I hergestellt werden, indem man die nach bekannten Verfahren zugänglichen 1-Aryl-4-(2-nitroaryl)-1,4-butadiene

(Tetrahedron Lett. 1983, 1441) der allgemeinen Formel II in der $R^1$, $R^2$ und $R^3$ die oben genannte Bedeutung haben, durch Cycloaddition mit Maleinimid zu Verbindungen der allgemeinen Formel III umsetzt. Für den Fall, daß entweder $R^1$ oder $R^2$ eine 2-Nitrogruppe bedeutet, ist diese Reaktion bekannt (Tetrahedron Lett. 1983, 1441). Die Verbindungen der allgemeinen Formel III werden nach gängigen Verfahren, z.B. mit 2,3-Dichlor-5,6-dicyan-p-benzochinon (DDQ), Schwefel oder Palladium auf Aktivkohle, zu Verbindungen IV dehydriert. Durch Erhitzen der Verbindungen IV mit Phosphor(III)-Verbindungen, wie Triphenylphosphin oder Triethylphosphit in einem geeigneten inerten Lösungsmittel (Quart. Reviews 1968, 22, 222 ; Synthesis 1969, 11) werden neue Pyrrolocarbazol-Derivate Ia erhalten.

**Reaktionsschema I:**

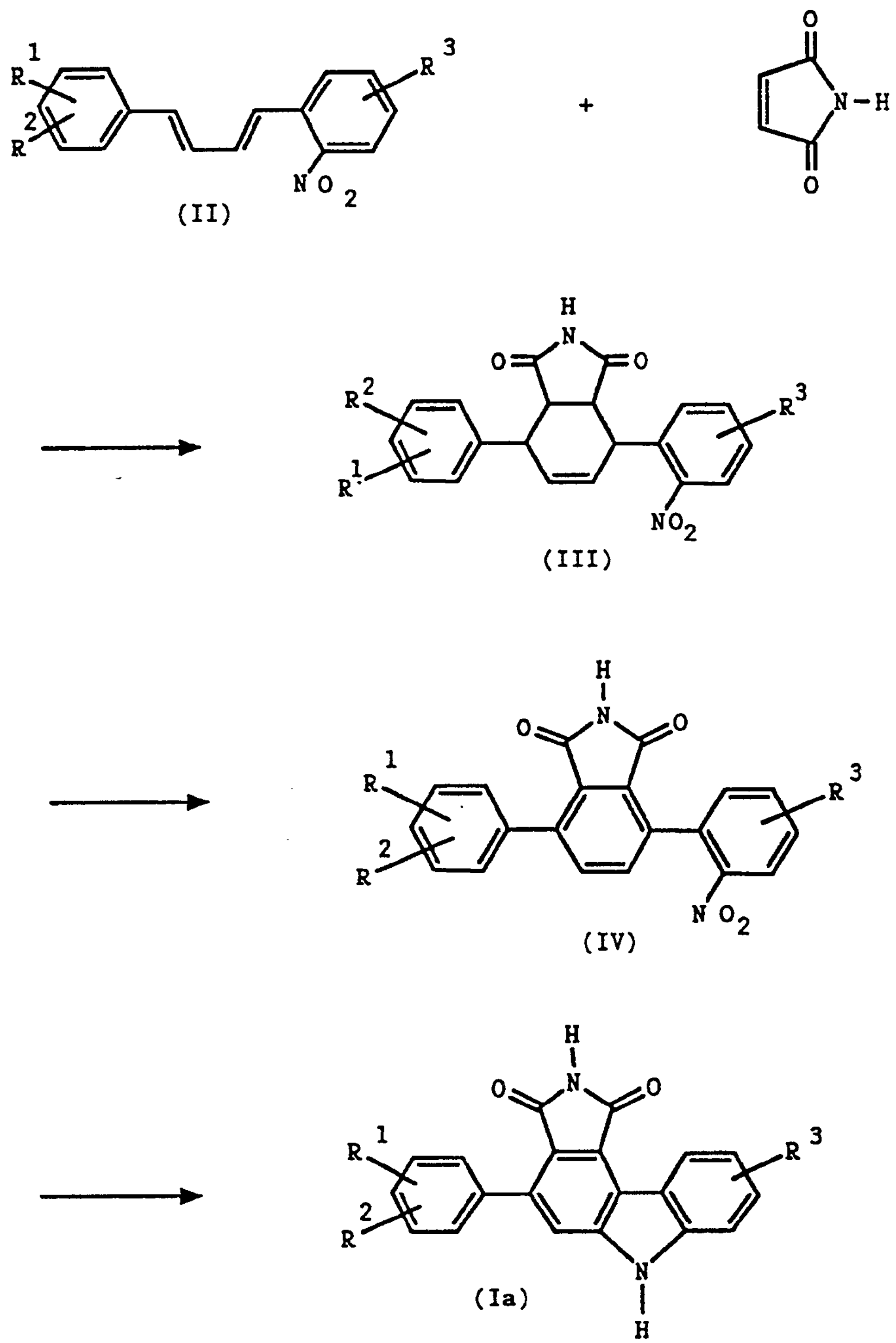

(II) (III) (IV) (Ia)

B) Verbindungen der allgemeinen Formel I, bei denen sowohl A und B als auch C und D ein Carbonylsauerstoffatom bilden und $R^1$, $R^2$, $R^3$ und $R^4$ die oben angegebenen Bedeutungen besitzen (Verbindungen Ib), können gemäß Reaktionsschema II hergestellt werden, indem man unsubstituierte oder substituierte Indol-2-aldehyde oder am Indolstickstoffatom substituierte Derivate der allgemeinen Formel V, in der $R^3$ und $R^4$ die oben angegebene Bedeutung besitzen, durch eine Wittig-Reaktion mit geeignet substituierten Arylmethyltriphenylphosphoniumhalogeniden der allgemeinen Formel VI, in der $R^1$ und $R^2$ die oben angegebene Bedeutung haben und X Halogen, vorzugsweise Chlor oder Brom bedeutet, in Gegenwart geeignete Basen, wie z.B. Alkalicarbonaten, -alkoholaten, -amiden oder lithiumorganischen Verbindungen, zu 2-(2-Arylvinyl)indolen der allgemeinen Formel VII umsetzt. Andere geeignete Verfahren zur Herstellung von Verbindungen der allgemeinen Formel VII sind beschrieben (Chem. Commun. 1970, 1095 ; Can. J. Chem. 1973, 792 ; Tetrahedron Lett. 1984, 3101). Die Verbindungen der allgemeinen Formel VII werden je nach Reaktionsbedingungen als cis-/trans- Isomerengemische erhalten, die durch übliche Verfahren wie Kristallisation oder Chromatographie getrennt werden können.

Verbindungen der allgemeinen Formel VII, bei denen $R^4$ für eine der oben definierten Aminoalkylgruppen mit 1 bis 12 C-Atomen oder eine Cyanoalkylgruppe mit 2 bis 4 C-Atomen steht, werden vorzugsweise durch Aminoalkylierung oder Cyanoalkylierung von Verbindungen der allgemeinen Formel VII, bei denen $R^4$ für Wasserstoff steht, nach bekannten Verfahren der Aminoalkylierung oder Cyanoalkylierung von Indolderivaten hergestellt.

Verbindungen der allgemeinen Formel VII, bei denen $R^4$ für eine 2-Cyanoethylgruppe steht, werden vorzugsweise durch Michaeladdition von Verbindungen der allgemeinen Formel VII, bei denen $R^4$ für Wasserstoff steht, an Acrylnitril, in Gegenwart eines basischen Katalysators (z.B. 1,8-Diazabicyclo[5,4,o]undec-7-en = DBU), in an sich bekannter Weise hergestellt (vgl. J. Med. Chem. 1989, 32, 73).

Verbindungen der allgemeinen Formel VII, bei denen $R^4$ für eine 2-Alkoxycarbonylethylgruppe mit bis zu 7 C-Atomen steht, werden vorzugsweise durch Michaeladdition von Verbindungen der allgemeinen Formel VII, bei denen $R^4$ für Wasserstoff steht, an Acrylsäureester mit bis zu 7 C-Atomen, in Gegenwart eines basischen Katalysators (z.B. 1,8-Diazabicyclo[5,4,o]undec-7-en = DBU), in an sich bekannter Weise hergestellt.

Die trans-Isomeren von Verbindungen der allgemeinen Formel VII werden durch Erhitzen mit Maleinimid in einem geeigneten Lösungsmittel in die Verbindungen der allgemeinen Formel VIII übergeführt. Die cis-Isomeren von Verbindungen der allgemeinen Formel VII oder Isomerengemische werden unter Zusatz von Katalysatoren wie Aluminiumtrichlorid mit Maleinimid in einem geeigneten Lösungsmittel in die Verbindungen der allgemeinen Formel VIII übergeführt. Cycloadditionen von 2-Vinylindolen mit geeigneten Dienophilen, u.a. auch mit substituierten Maleinimiden sind beschrieben (Heterocycles 1988, 1253 ; Heterocycles 1988, 967 ; Can. J. Chem. 1982, 419). Die Verbindungen der allgemeinen Formel VIII werden nach gängigen Verfahren, z.B. mit 2,3-Dichlor-5,6-dicyan-p-benzochinon, Palladium auf Aktivkohle, Schwefel oder Natriumnitrit in Eisessig zu den neuen Pyrrolocarbazol-Derivaten der allgemeinen Formel Ib dehydriert.

**Reaktionsschema II:**

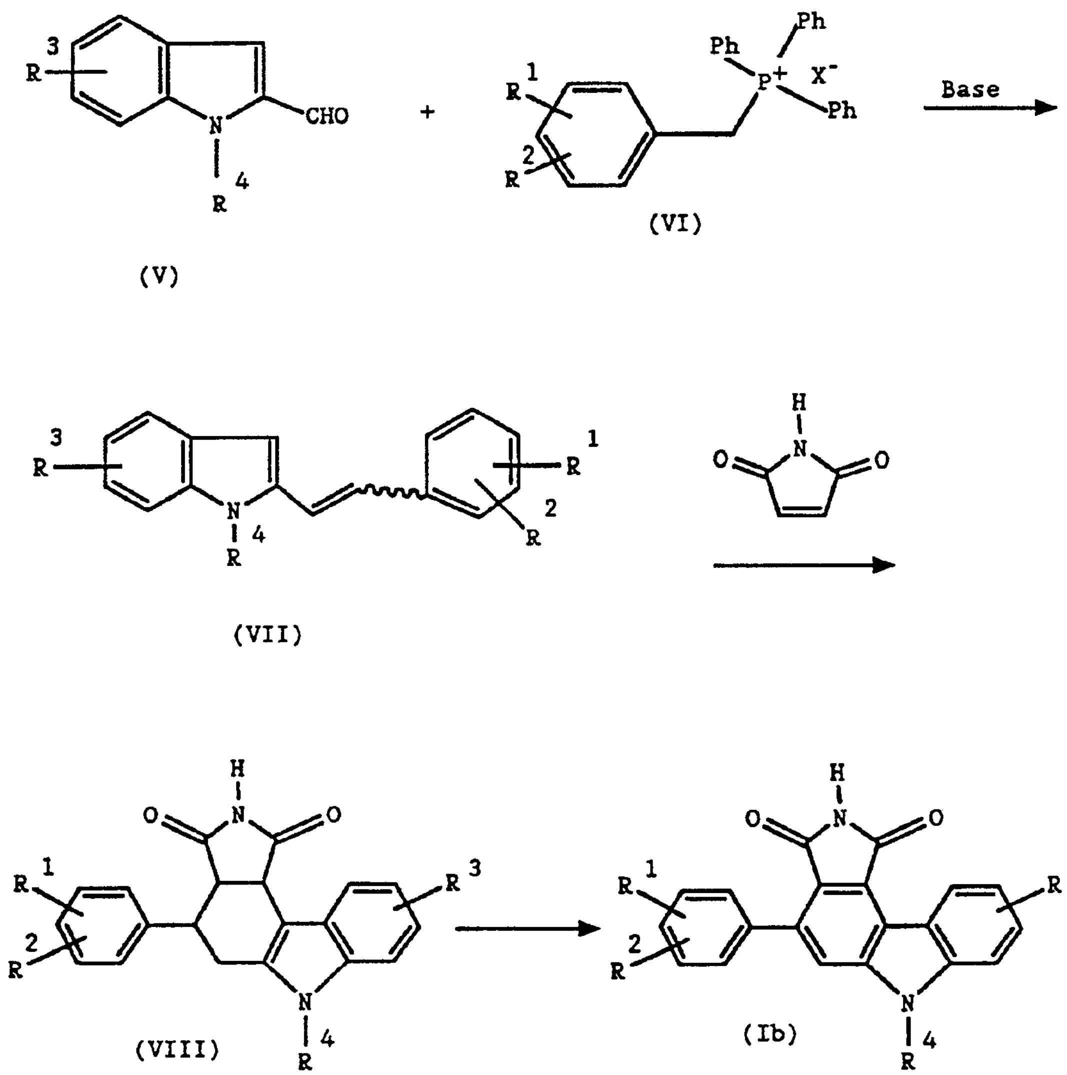


(V) (VI) (VII) (VIII) (Ib)

C) Lactame der allgemeinen Formel I, bei denen entweder A und B oder C und D für Wasserstoff stehen und die anderen beiden Reste zusammen ein Carbonylsauerstoffatom bilden (Verbindungen Ic) werden durch Reduktion von Imiden der allgemeinen Formeln Ia oder Ib, bei denen sowohl A und B als auch C und D ein Carbonylsauerstoffatom bilden, gemäß Reaktionsschema III hergestellt. Als bevorzugte Reduktionsmittel werden Zinkamalgam/Chlorwasserstoffgas in C1-C4-Alkoholen oder Zinkamalgam in Eisessig oder Zink in Eisessig verwendet. Die teilweise anfallenden Regioisomerengemische von Verbindungen Ic können durch übliche Verfahren wie Kristallisation oder Chromatographie getrennt Werden. Vorzugsweise wird unter den angegebenen Reaktionsbedingungen das Carbonylsauerstoffatom, das durch C und D gebildet wird, reduziert.

Reaktionsschema III:

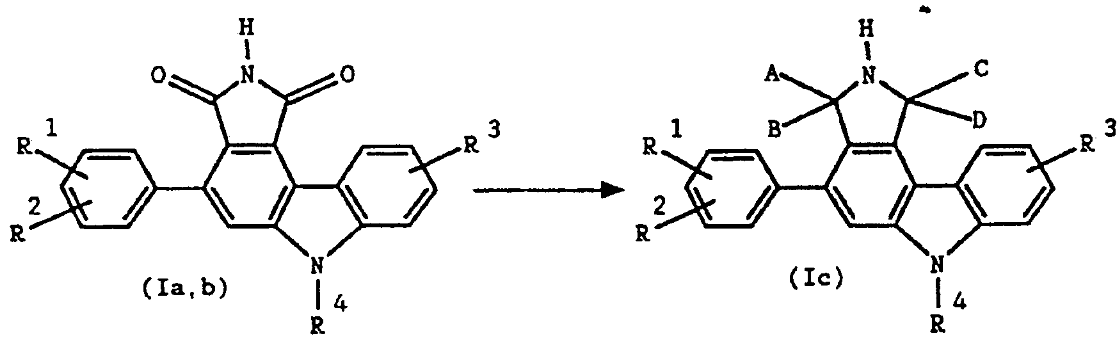

D) Hydroxylactame der allgemeinen Formel I, bei denen entweder A und B oder C und D zusammen ein Carbonylsauerstoffatom bilden, einer der beiden anderen Reste für Wasserstoff und der andere für eine Hydroxygruppe steht (Verbindungen Id) werden gemäß Reaktionsschema IV ebenfalls durch Reduktion von Imiden der allgemeinen Formeln Ia oder Ib hergestellt. Als bevorzugte Reduktionsmittel werden Zinkamalgam/Chlorwasserstoffgas in C1-C4-Alkoholen bei Temperaturen unter 20°C oder Borhydride, wie z.B. Natriumborhydrid, vorzugsweise in C1-C4-Alkoholen oder Alkohol/Wasser-Gemischen oder Lithiumaluminiumhydrid in einem aprotischen Lösungsmittel eingesetzt. Die teilweise anfallenden Regioisomerengemische von Verbindungen Id können durch übliche Verfahren der Kristallisation oder Chromatographie getrennt werden.

Reaktionsschema IV:

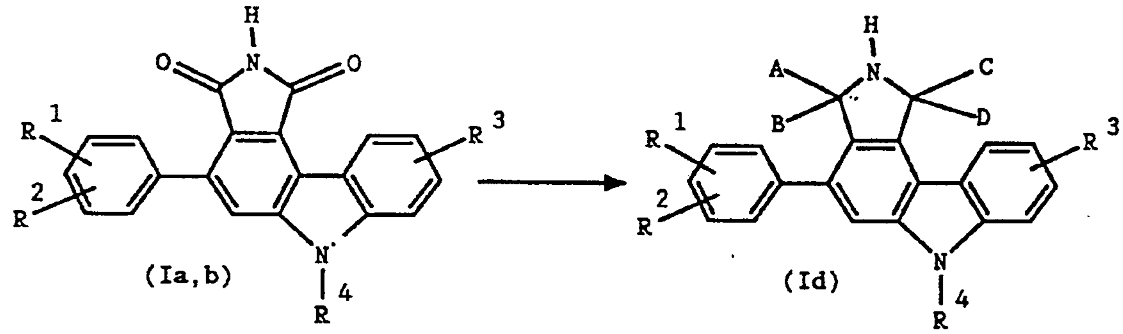

E) Lactame der allgemeinen Formel Ic, bei denen $R^4$ für Wasserstoff steht (Verbindungen Ic') können durch Umsetzung mit Verbindungen $R^{4'}$-X, bei denen $R^{4'}$ mit Ausnahme von Wasserstoff die für $R^4$ angegebenen Bedeutungen besitzt und X vorzugsweise für Halogen, insbesondere Jod, Brom oder Chlor, steht, gemäß Reaktionsschema V in Gegenwart von Basen wie Hydrigen, Carbonaten, Hydroxiden, Oxiden oder Alkoxiden der Alkali- oder Erdalkalimetalle, oder von lithiumorganischen Verbindungen in an sich bekannter Weise am Indolstickstoffatom zu Lactamen der allgemeinen Formel Ic'' alkyliert werden. Das beschriebene Verfahren der Alkylierung der Verbindungen Ic' ist überraschend, da nicht absehbar war, daß die Einführung von dem Rest $R^{4'}$ selektiv am Indol-Stickstoffatom und nicht am Stickstoffatom des Lactamrings erfolgt.

Reaktionsschema V:

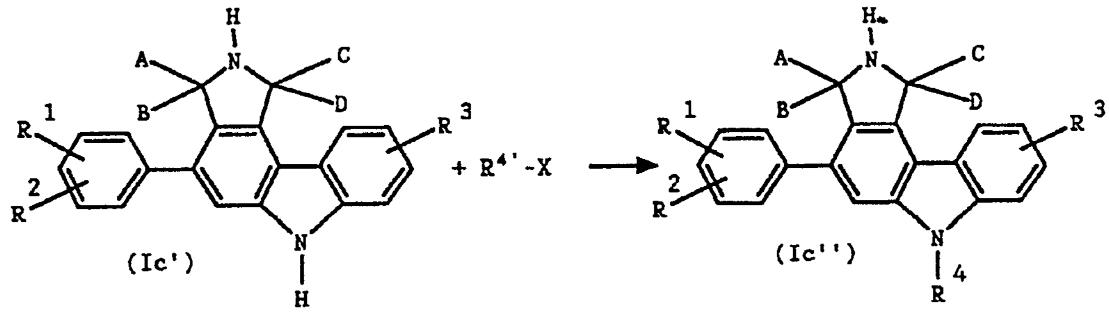

F) Verbindungen der allgemeinen Formel I, bei denen $R^1$ und/oder $R^2$ und/oder $R^3$ für eine Hydroxygruppe stehen, können auch in an sich bekannter Weise durch Etherspaltung von Verbindungen der allgemeinen Formel I, bei denen $R^1$ und/oder $R^2$ und/oder $R^3$ für eine Alkoxygruppe mit 1 bis 4 C-Atomen stehen, hergestellt werden.

Verbindungen der allgemeinen Formel I, bei denen $R^1$ und/oder $R^2$ und/oder $R^3$ für eine unsubstituierte Aminogruppe stehen, können auch in an sich bekannter Weise durch Reduktion von Verbindungen der allgemeinen Formel I, bei denen $R^1$ und/oder $R^2$ und/oder $R^3$ für eine Nitrogruppe stehen, hergestellt werden.

Verbindungen der allgemeinen Formel I, bei denen $R^1$ und/oder $R^2$ und/oder $R^3$ für eine unsubstituierte oder substituierte Aminoalkoxygruppe stehen, können auch in an sich bekannter Weise durch Aminoalkylierung von Verbindungen der allgemeinen Formel I, bei denen $R^1$ und/oder $R^2$ und/oder $R^3$ für eine Hydroxygruppe stehen, hergestellt werden. Die dabei eingesetzten Aminoalkylreste entsprechen denen, die im nachfolgenden Abschnitt als für $R^4$ besonders geeignet definiert werden.

Für $R^4$ besonders geeignete unsubstituierte oder substituierte Aminoalkylgruppen mit bis zu 12 C-Atomen sind unsubstituierte Aminoalkylgruppen, wie ein 2-Aminoethyl-, ein 3-Aminopropyl- oder ein 1-Amino-2-propylrest, N,N-Dialkylaminoalkyl- oder N,N-Alkylbenzylaminoalkylgruppen mit C1-C4-Alkylsubstituenten an den Stickstoffatomen und 1-4 C-Atomen in der Alkylkette, wobei die Alkylketten durch weitere C1-C4-Alkylreste, eine Hydroxy- oder eine Methoxygruppe substituiert sein können, insbesondere eine 2-Dimethylaminoethyl-, 3-Dimethylamino-1-propyl-, 3-Dimethylamino-2-propyl-, 2-Diethylaminoethyl-, 2-[N-Benzyl-N-methylamino]ethyl-, 3-[N-Benzyl-N-methylamino]propyl-, 3-Dimethylamino-2-hydroxy-1-propyl-, 3-Diethylamino-2-hydroxy-1-propyl-, 2-Piperidinoethyl-, 3-Piperidinopropyl-, 2-Pyrrolidinoethyl-, 3-Pyrrolidinopropyl-, 2-Morpholinoethyl-, 3-Morpholinopropyl-, Piperidin-2-ylmethyl-, N-Methyl-piperidin-2-ylmethyl-, 3-Dimethylamino-2-methoxy-1-propyl-, 3-Diethylamino- 2-methoxy-1-propyl-, Pyrrolidin-2-ylmethyl- oder eine N-Methylpyrrolidin-2-ylmethylgruppe. Weitere für $R^4$ besonders geeignete Reste sind geradkettige oder verzweigte Alkylgruppen mit 1 bis 4 C-Atomen, insbesondere Methyl, Ethyl, n-Propyl, Isopropyl und n-Butyl, Cyanoalkylgruppen mit 2 oder 3 C-Atomen, insbesondere Cyanomethyl und 2-Cyanoethyl, Alkoxycarbonylalkylgruppen mit bis zu 7 C-Atomen, insbesondere 2-Methoxycarbonylethyl, 2-Ethoxycarbonylethyl und Methoxycarbonylmethyl.

Für $R^1$ und $R^2$ bevorzugte Restekombinationen sind :

$R^1$ und $R^2$ Wasserstoff oder $R^1$ Wasserstoff und $R^2$ eine 2-Methyl-, 2-Chlor-, 3-Chlor-, 4-Chlor-, 2-Brom-, 2-Fluor-, 2-Trifluormethyl-, 2-Methoxy-, 3-Methoxy-, 4-Methoxy, 2-Amino-, 2-Nitro-, 2-Hydroxy- oder 2-(3-Dimethylaminopropoxy)-gruppe oder $R^1$ eine 2-Nitrogruppe und $R^2$ eine 5-Methoxygruppe:

Lactame oder Hydroxylactame der allgemeinen Formel I, bei denen die Reste A und B verschieden von den Resten C und D sind, können auch als Regioisomerengemische verwendet werden, oder wie bereits beschrieben, durch bekannte Verfahren wie Kristallisation oder Chromatographie getrennt werden.

Hydroxylactame der allgemeinen Formel I, bei denen einer der Reste A, B, C oder D eine Hydroxygruppe bedeutet und an ein chirales Zentrum gebunden ist, oder Verbindungen der allgemeinen Formel I, die in den Resten $R^1$, $R^2$, $R^3$ oder $R^4$ ein chirales Zentrum aufweisen, können als Stereoisomerengemische oder in Form der Enantiomeren verwendet werden. Die Enantiomeren können nach den für optische Trennungen von Stereoisomeren verwendeten üblichen Verfahren erhalten werden.

Basische Verbindungen der allgemeinen Formel I, welche ein basisches Zentrum an $R^1$, $R^2$, $R^3$ oder $R^4$ aufweisen, werden zum Zwecke der Reinigung und aus galenischen Gründen bevorzugt in kristalline, pharmakologisch verträgliche Salze übergeführt. Die Salze werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Fumarsäure, Oxalsäure oder Bernsteinsäure in Frage. Die Säureadditionssalze werden in der Regel in an sich bekannter Weise durch Mischen der freien Base oder deren Lösungen mit der entsprechenden Säure oder deren Lösungen in einem organischen Lösunsgsmittel, beispielsweise einem niederen Alkohol wie Methanol, Ethanol oder 2-Propanol oder einem niederen Keton wie Aceton oder 2-Butanon oder einem Ether wie Diethylether, Diisopropylether, Tetrahydrofuran oder Dioxan, erhalten.

Die erfindungsgemäßen Verbindungen sind potente Inhibitoren von Proteinkinasen wie der Proteinkinase C. So zeigt z.B. die Verbindung von Beispiel 2.a im Enzym-Assay der mit Phosphatidylserin und Diacylglycerol aktivierten Proteinkinase C eine 50%ige Inhibierung bei einer Konzentration von 0,58 µmol/l. Der Versuch wurde gemäß EP-OS-0 255 126 (Hemmung von Proteinkinase C) durchgeführt.

Die Proteinkinase C spielt für die intracelluläre Signaltransduktion eine wichtige Schlüsselrolle und ist eng mit der Regulation von kontraktilen, sekretorischen und proliferativen Prozessen verknüpft. Aufgrund dieser Eigenschaften können die erfindungsgemäßen Verbindungen zur Prävention und/oder Behandlung von Herz- und Gefäßkrankheiten wie Thrombosen, Arteriosklerose, Hypertension, von Entzündungsprozessen, Allergien, Krebs und bestimmten degenerativen Schäden des Zentralnervensystems verwendet werden, sowie zur Behandlung viraler Erkrankungen. Die Verbindungen können in der jeweils geeigneten Formulierung enteral oder parenteral in Dosen von 1 bis 500 mg/kg, bevorzugt 1 bis 50 mg/kg verabreicht werden.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z. B. Tartrat- und Citrat-Puffer, Ethanol, Komplexbildner (wie Äthylendiamin-tetraessigsäure und deren nicht-toxische Salze) sowie hochmolekulare Polymere (wie flüssiges Polyäthylenoxid) zur Viskositätsregulierung. Feste Trägerstoffe sind z. B. Särke, Lactose, Mannit, Methylcellulose, Talkum, hochdisperse Kieselsäuren, höhermolekulare Fettsäuren (wie Stearinsäure), Gelatine, Agar-Agar, Calciumphosphat Magnesiumstearat, tierische und pflanzliche Fette, feste hochmolekulare Polymere (wie Polyäthylenglykol) ; für orale Applikation geeignete Zubereitungen können gewünschtenfalls zusätzliche Geschmacks- und/oder Süßstoffe enthalten.

Die folgenden Beispiele dienen der näheren Erläuterung der Erfindung :

## Beispiele

Beispiel 1 (nach Verfahren A)

1,2,3,6-Tetrahydro-1,3-dioxo-4-phenyl-pyrrolo[3,4-c]carbazol

3,5 g (10,2 mmol)1,3-Dihydro-4-(2-nitrophenyl)-1,3-dioxo-7-phenyl-2H-isoindol und 9,5 g (36,2 mmol) Triphenylphosphin werden in 100 ml 2,4,6-Collidin 16 h unter Rückfluß erhitzt. Das Lösungsmittel wird im Vakuum abdestilliert und der Rückstand an Kieselgel mit Toluol/Ethylacetat 3 : 1 chromatographiert. Die Fraktion mit dem Rf 0.3 wird isoliert und aus Cyclohexan/Aceton umkristallisiert. Man erhält 1,2,3,6-Tetrahydro-1,3-dioxo-4-phenyl-pyrrolo[3,4-c]-carbazol in Form gelber Kristalle vom Schmp. 262-264°C.

Das als Ausgangsprodukt verwendete 1,3-Dihydro-4-(2-nitrophenyl)-1,3-dioxo-7-phenyl-2H-isoindol wird wie folgt hergestellt :

5,5 g (15,8 mmol) 1,3,3a,4,7,7a-Hexahydro-4-(2-nitrophenyl)-1,3-dioxo-7-phenyl-2H-isoindol und 9,1 g (40,1 mmol) 2,3-Dichlor-5,6-dicyan-p-benzochinon (DDQ) werden in 100 ml t-Butylbenzol 16 h unter Rückfluß erhitzt. Das Lösungsmittel wird im Vakuum entfernt und der Rückstand an Kieselgel mit Toluol/Ethylacetat 3 : 1 chromatographiert. Die Fraktion mit dem Rf 0.4 wird isoliert, mit wenig heißem Ethanol verrührt und die nach dem Abkühlen gebildeten Kristalle abfiltriert. Nach Umkristallisation aus Ethylacetat erhält man 1,3-Dihydro-4-(2-nitrophenyl)-1,3-dioxo-7-phenyl-2H-isoindol in Form farbloser Kristalle vom Schmp. 224-226°C.

Das als Ausgangsprodukt verwendete 1,3,3a,4,7,7a-Hexahydro-4-(2-nitrophenyl)-1,3-dioxo-7-phenyl-2H-isoindol wird wie folgt hergestellt :

7,5 g (29,8 mmol) (E, E)-1-(2-Nitrophenyl)-4-phenyl-1,3-butadien und 2,9 g (29,9 mmol) Maleinsäureimid werden in 10 ml Toluol 12 h auf 130°C erhitzt. Nach Zugabe von 1,5 g (15,5 mmol) Maleinsäureimid wird weitere 24 h bei 130°C erhitzt. Nach dem Abkühlen wird mit 40 ml Dichlormethan verdünnt, das Produkt abfiltriert und aus Ethanol kristallisiert. Man erhält 1,3,3a,4,7,7a-Hexahydro-4-(2-nitrophenyl)-1,3-dioxo-7-phenyl-2H-isoindol in Form blaßgelber Kristalle, die sich von 200-208°C zersetzen.

Das als Ausgangsprodukt verwendete (E, E)-1-(2-Nitrophenyl)-4-phenyl-1,3-butadien wird in Analogie zu Tetrahedron Lett. 1983, 1441 durch Isomerisierung mit Jod in Toluol des bei der Umsetzung von 2-Nitrobenzyltriphenylphosphoniumbromid mit trans-Zimtaldehyd gebildeten (E, E)- und (E, Z)-Isomerengemisches erhalten.

In analoger Weise wird die folgende Verbindung erhalten :

Beispiel 1.a :

1,2,3,6-Tetrahydro-4-(2-methoxyphenyl)-1,3-dioxo-pyrrolo-[3,4-c]-carbazol Schmp. > 300°C (Zers.) aus Toluol (ausgehend von 2-Nitrobenzyltriphenylphosphoniumbromid und 2-Methoxyzimtaldehyd)

Beispiel 1 (nach Verfahren B)

1,2,3,6,Tetrahydro-1,3-dioxo-4-phenyl-pyrrolo[3,4-c]carbazol

1,2 g (3,8 mmol) 1,2,3,3a,4,5,6,10c-Octahydro-1,3-dioxo-4-phenyl-pyrrolo[3,4-c]carbazol und 2,2 g (9,7 mmol) DDQ werden in 30 ml Toluol 20 h unter Rückfluß erhitzt. Das Lösungsmittel wird im Vakuum abdestilliert und der Rückstand an Kieselgel mit Toluol/Ethylacetat 3 : 1 chromatographiert. Die Fraktion mit dem Rf 0.3 wird isoliert, mit Aceton/Diisopropylether ausgerührt und die gebildeten Kristalle abfiltriert. Man erhält 1,2,3,6-Tetrahydro-1,3-dioxo-4-phenyl-pyrrolo[3,4-c]carbazol in Form gelber Kristalle vom Schmp. 262-263°C.

Das als Ausgangsprodukt verwendete 1,2,3,3a,4,5,6,10c-Octahydro-1,3-dioxo-4-phenyl-pyrrolo[3,4-c]carbazol wird wie folgt hergestellt :

1,3 g (5,9 mmol) (E)-1-(2-Indolyl)-2-phenylethylen und 0,7 g (7,2 mmol) Maleinsäureimid werden in 25 ml Toluol 16 h unter Rückfluß erhitzt. Nach Zugabe von 0,1 g (1 mmol) Maleinsäureimid wird weitere 8 h refluxiert. Nach dem Abkühlen werden die gebildeten Kristalle abfiltriert und an Kieselgel mit Toluol/Ethylacetat 4 : 1 chromatographiert. Die Fraktion mit dem Rf 0.1 wird isoliert und aus Toluol umkristallisiert. Man erhält 1,2,3,3a,4,5,6,10c-Octahydro-1,3-dioxo-4-phenyl-pyrrolo[3,4-c]carbazol in Form farbloser Kristalle vom Schmp. 212-215°C.

Das als Ausgangsprodukt verwendete (E)-1-(2-Indolyl)-2-phenylethylen wird wie folgt hergestellt :

2,0 g (13,7 mmol) 2-Indolaldehyd und 5,3 g (13,7 mmol) Benzyltriphenylphosphoniumchlorid werden in 30 ml trockenem Ethanol zum Sieden erhitzt. Innerhalb von ca. 1.5 h wird eine frisch bereitete Lösung von 0,32 g (13,9 mmol) Natrium in 15 ml Ethanol zugetropft. Anschließend wird 1 h unter Rückfluß gekocht, das Lösungsmittel nach dem Abkühlen abdestilliert und der Rückstand zwischen Dichlormethan und Wasser verteilt. Die organische Phase wird über Natriumsulfat getrocknet, eingedampft und der Rückstand an Kieselgel mit Cyclohexan/Toluol 7 : 3 chromatographiert. Die Fraktion mit dem Rf 0.4 wird isoliert. Man erhält (Z)-1-(2-Indolyl)-2-phenylethylen in Form eines blaßgelben Öls, das beim Trocknen im Vakuum kristalliert. Die Fraktion mit dem Rf 0.2 wird eingedampft, der Rückstand mit Cyclohexan verrührt und die gebildeten Kristalle abfiltriert. Man erhält (E)-1-(2-Indolyl)-2-phenylethylen in Form nahezu farbloser Kristalle vom Schmp. 205-206°C. Die Darstellung des 2-Indolaldehyds erfolgt nach Literaturangaben (Arch. Pharm. 310, 975, 1977) aus 2-Indolcarbonsäureestern.

In analoger Weise werden die folgenden Beispiele erhalten. Soweit diese am Carbazolstickstoffatom substituiert sind, wurde der entsprechende Rest $R^4$ vorzugsweise bereits durch Alkylierung der entsprechenden 2-Indolcarbonsäureester nach bekannten Verfahren der N-Alkylierung von Indolen (z.B. analog Synthesis 1976, 124) eingeführt :

Beispiel 2. :

6-Ethyl-1,2,3,6-tetrahydro-1,3-dioxo-4-phenyl-pyrrolo[3,4-c]carbazol Schmp. 272-277°C aus Toluol

Beispiel 2.a :

4-(2-Chlorphenyl)-6-ethyl-1,2,3,6-tetrahydro-1,3-dioxo-pyrrolo[3,4-c]carbazol Schmp. 272-274°C aus Toluol

Beispiel 2.b :

4-(4-Chlorphenyl)-6-ethyl-1,2,3,6-tetrahydro-1,3-dioxo-pyrrolo[3,4-c]carbazol Schmp. > 300°C aus Toluol

Beispiel 2.c :

4-(3-Chlorphenyl)-6-ethyl-1,2,3,6-tetrahydro-1,3-dioxo-pyrrolo[3,4-c]carbazol Schmp. 298-299°C aus

Toluol/Diisopropylether

Beispiel 2.d :

4-(2-Chlorphenyl)-1,2,3,6-tetrahydro-6-methyl-1,3-dioxo-pyrrolo[3,4-c]carbazol Schmp. 250-255°C (Zers.) aus Diisopropylether

Beispiel 2.e :

1,2,3,6-Tetrahydro-6-methyl-4-phenyl-1,3-dioxo-pyrrolo[3,4-c]carbazol Schmp. 312-314°C aus Diisopropylether

Beispiel 2.f :

4-(2-Chlorphenyl)-1,2,3,6-tetrahydro-1,3-dioxo-pyrrolo[3,4-c] carbazol Schmp. 310-313°C aus Ethanol

Beispiel 2.g :

1,2,3,6-Tetrahydro-4-(2-nitrophenyl)-1,3-dioxo-pyrrolo[3,4-c]carbazol Schmp. 310-312°C aus Ethylacetat

Beispiel 2.h :

4-(4-Chlorphenyl)-1,2,3,6-tetrahydro-6-methyl-1,3-dioxo-pyrrolo[3,4-c]carbazol Schmp. 332-337°C aus Ethanol

Beispiel 2.i :

4-(3-Chlorphenyl)-1,2,3,6-tetrahydro-6-methyl-1,3-dioxo-pyrrolo[3,4-c]carbazol Schmp. 299-302°C aus Ethanol

Beispiel 2.j :

4-(2-Trifluormethylphenyl)-1,2,3,6-tetrahydro-6-methyl-1,3-dioxo-pyrrolo[3,4-c]carbazol Schmp. 310-312°C aus Dichlormethan.

Beispiel 3 (nach Verfahren C) :

1,2,3,6-Tetrahydro-4-(2-methoxyphenyl)-3-oxo-pyrrolo[3,4-c]carbazol

5 g (76 mmol) Zinkpulver und 0,5 g (1,8 mmol) Quecksilber(II)chlorid in 5 ml Wasser werden 20 min bei 20°C gerührt, dann 0,15 ml konz. Salzsäure zugefügt und 1 min weitergerührt. Sofort danach wird das Zinkpulver zunächst mit Wasser, dann mit Ethanol und zum Schluß mit getrocknetem Ethanol gewaschen. Das Zinkpulver wird in 100 ml trockenem Ethanol suspendiert. Unter Rühren werden 1,2 g (3,5 mmol) 1,2,3,6-Tetrahydro-4-(2-methoxy)phenyl-1,3-dioxo-pyrrolo[3,4-c]carbazol (Beispiel 1.a) zugefügt und es wird bei langsamem Durchleiten von getrocknetem Chlorwasserstoff 3 h unter Rückfluß erhitzt. Nach dem Abkühlen wird eingedampft und der Rückstand zwischen Kaliumcarbonatlösung und Ethylacetat verteilt. Die wäßrige Phase wird erneut mit Ethylacetat extrahiert, die vereinten organischen Phasen über Natriumsulfat getrocknet und eingedampft. Der Rückstand wird an Kieselgel mit Toluol/Ethylacetat 1 : 1 chromatographiert, die Fraktion mit dem Rf 0.15 isoliert, mit Toluol/Ethanol 9 : 1 verrührt und die gebildeten Kristalle abfiltriert. Man erhält 1,2,3,6-Tetrahydro-4-(2-methoxyphenyl)-3-oxo-pyrrolo[3,4-c]carbazol in Form blaßgelber Kristalle, die sich zwischen 265 und 268°C zersetzen.

In analoger Weise werden die folgenden Verbindungen erhalten :

Beispiel 3.a :

1,2,3,6-Tetrahydro-3-oxo-4-phenyl-pyrrolo[3,4-c]carbazol Schmp. ~250°C (Zers.) aus Diisopropylesther (hergestellt aus Beispiel 1)

Beispiel 3.b :

4-(2-Chlorphenyl)-6-ethyl-1,2,3,6-tetrahydro-3-oxo-pyrrolo[3,4-c]carbazol Schmp. 265-275°C (Zers.) aus Diisopropylether (hergestellt aus Beispiel 2.a)

Beispiel 4 (nach Verfahren D) :

4-(2-Chlorphenyl)-6-ethyl-1,2,3,6-tetrahydro-1-hydroxy-3-oxo-pyrrolo[3,4-c]carbazol (Beispiel 4.a) und

4-(2-Chlorphenyl)-6-ethyl-1,2,3,6-tetrahydro-3-hydroxy-1-oxo-pyrrolo[3,4-c]carbazol (Beispiel 4.b)

0,8 g (2,13 mmol) 4-(2-Chlorphenyl)-6-ethyl-1,2,3,6-tetrahydro-1,3-dioxo-pyrrolo[3,4-c]carbazol (Beispiel 2.a) werden in 20 ml 90%igem Methanol suspendiert, unter kräftigem Rühren eine Lösung von 0,16 g (4,23 mmol) Natriumborhydrid in 5 ml Methanol zugetropft und 5 d bei Raumtemperatur gerührt. Das überschüssige Natriumborhydrid wird mit Essigsäure zersetzt, das Lösungsmittel abdestilliert und der kristalline Rückstand an Kieselgel mit Toluol/Ethylacetat 1 : 1 chromatographiert. Die Fraktion mit dem Rf 0.3 wird isoliert, mit Toluol/Diisopropylether verrührt und die gebildeten Kristalle werden abfiltriert. Man erhält 4-(2-Chlorphenyl)-6-ethyl-1,2,3,6-tetrahydro-1-hydroxy-3-oxo-pyrrolo[3,4-c]carbazol (Beispiel 4.a) in Form blaßbeiger Kristalle vom Schmp. 225-228°C.

Die Fraktion mit dem Rf 0.25 (Toluol/Ethylacetat 1 : 1) wird isoliert, mit Diisopropylether verrührt und die gebildeten Kristalle abfiltriert. Man erhält 4-(2-Chlorphenyl)-6-ethyl-1,2,3,6-tetrahydro-3-hydroxy-1-oxo-pyrrolo[3,4-c]carbazol (Beispiel 4.b) in Form farbloser Kristalle vom Schmp. 232-236°C.

Beispiel 5 :

1,2,3,6-Tetrahydro-4-(2-hydroxyphenyl)-3-oxo-pyrrolo[3,4-c]carbazol

Zu 212 mg (0,65 mmol) 1,2,3,6-Tetrahydro-4-(2-methoxyphenyl)-3-oxo-pyrrolo[3,4-c]carbazol in 25 ml trockenem Dichlormethan bei −78°C wird eine Lösung von 0,1 ml (1,06 mmol) Bortribromid in 2 ml Dichlormethan getropft. Nach 1 h bei −78°C wird auf Raumtemperatur erwärmt und weitere 1,5 h bei Raumtemperatur gerührt. Es wird auf −10°C gekühlt und 10 ml Wasser zugetropft. Nach dem Erwärmen auf Raumtemperatur wird mit Ethylacetat verdünnt und der in beiden Phasen unlösliche Niederschlag abfiltriert. Die organische Phase des Filtrats wird abgetrennt, über Natriumsulfat getrocknet und eingedampft. Der Rückstand wird zusammen mit dem abfiltrierten unlöslichen Niederschlag mit Ethylacetat ausgekocht. Nach dem Abkühlen werden die Kristalle abfiltriert. Man erhält 1,2,3,6-Tetrahydro-4-(2-hydroxyphenyl)-3-oxo-pyrrolo[3,4-c]carbazol in Form blaßbeiger Kristalle vom Schmp. > 300°C (Zers.).

Beispiel 6 :

4-[2-(3-Dimethylaminopropoxy)phenyl]-6-(3-dimethylaminopropyl)-1,2,3,6-tetrahydro-3-oxo-pyrrolo [3,4-c] carbazol · Dihydrochlorid

98 mg (0,31 mmol) 1,2,3,6-Tetrahydro-4-(2-hydroxyphenyl)-3-oxo-pyrrolo[3,4-c]carbazol (Beispiel 5) werden mit 80 mg (0,66 mmol) 3-Dimethylaminopropylchlorid, 91 mg (0,66 mmol) Kaliumcarbonat und einer Spatelspitze Kaliumjodid in 25 ml Aceton 72 h unter Rückfluß erhitzt. Nach dem Abkühlen wird eingedampft und der Rückstand zwischen Wasser und Ethylacetat verteilt. Die organische Phase wird abgetrennt, über Natriumsulfat getrocknet und der nach dem Eindampfen erhaltene Rückstand an Kieselgel mit $NH_3$ gesättigtem Dichlormethan/Methanol 9: 1 chromatographiert. Die Fraktion mit dem Rf 0.6 wird isoliert, in einem Ether/Ethylacetat-Gemisch gelöst und mit Chlorwasserstoff in Ether das Dihydrochlorid gefällt. Man erhält 4-[2-(3-Dimethylaminopropoxy)phenyl]-6-(3-dimethylaminopropyl)-1,2,3,6-tetrahydro-3-oxo-pyrrolo[3,4-c]carb azol · Dihydrochlorid in Form beiger Kristalle, die sich oberhalb von 170°C zersetzen.

Beispiel 7 :

4-(2-Aminophenyl)-1,2,3,6-tetrahydro-1,3-dioxo-pyrrolo[3,4-c]-carbazol

0,54 g (1,51 mmol) 1,2,3,6-Tetrahydro-4-(2-nitrophenyl)-1,3-dioxo-pyrrolo[3,4-c]carbazol (Beispiel 2.g) in 30 ml Dimethylformamid werden mit 0,27 g Palladium auf Aktivkohle (10%) bei Raumtemperatur hydriert. Nach

4 h wird der Katalysator abfiltriert und die Lösung im Vakuum eingedampft. Der Rückstand wird mit Ethylacetat ausgerührt, die Kristalle abfiltriert und aus Methanol umkristallisiert. Man erhält 4-(2-Aminophenyl)-1,2,3,6-tetrahydro-1,3-dioxo-pyrrolo[3,4-c]carbazol in Form gelber Kristalle vom Schmp. > 300°C.

Beispiel 8 (nach Verfahren D) :

4-(4-Chlorphenyl)-6-ethyl-1,2,3,6-tetrahydro-1-hydroxy-3-oxo-pyrrolo[3,4-c]carbazol (Beispiel 8.a) und

4-(4-Chlorphenyl)-6-ethyl-1,2,3,6-tetrahydro-3-hydroxy-1-oxo-pyrrolo[3,4-c]carbazol (Beispiel 8.b)

Eine Lösung von 0,48 g (1,28 mmol) 4-(4-Chlorphenyl)-6-ethyl-1,2,3,6-tetrahydro-1,3-dioxo-pyrrolo[3,4-c]carbazol (Beispiel 2.b) in 35 ml trockenem Tetrahydrofuran wird bei 20°C zu einer Suspension von 0,097 g (2,56 mmol) Lithiumaluminiumhydrid in 10 ml Tetrahydrofuran getropft und 16 h bei Raumtemperatur gerührt. Das überschüssige Lithiumaluminiumhydrid wird mit Ethanol und Wasser zersetzt und der ausgefallene Niederschlag abfiltriert. Das Filtrat wird eingedampft und der Rückstand an Kieselgel mit Toluol/Ethylacetat 1 : 1 chromatographiert. Die Fraktion mit dem Rf 0.35 wird isoliert, mit Toluol/Ethylacetat verrührt und die Kristalle abfiltriert. Man erhält 4-(4-Chlorphenyl)-6-ethyl-1,2,3,6-tetrahydro-3-hydroxy-1-oxo-pyrrolo[3,4-c]carbazol (Beispiel 8.b) in Form gelber Kristalle vom Schmp. > 230°C (Zers.).

Die Fraktion mit dem Rf 0.25 wird isoliert, mit Toluol/Ethylacetat verrührt und die Kristalle abfiltriert. Man erhält 4-(4-Chlorphenyl)-6-ethyl-1,2,3,6-tetrahydro-1-hydroxy-3-oxo-pyrrolo[3,4-c]carbazol (Beispiel 8.a) in Form blaßgelber Kristalle vom Schmp. > 250°C (Zers.).

Beispiel 9 :

6-(3-Dimethylaminopropyl)-1,2,3,6-tetrahydro-1,3-dioxo-4-phenyl-pyrrolo[3,4-c]carbazol

5,89 (14,7 mmol) 6-(3-Dimethylaminopropyl)-1,2,3,3a,4,5,6,10c-octahydro-1,3-dioxo-4-phenyl-pyrrolo[3,4-c]carbazol in 150 ml Eisessig werden bei Durchleiten von Stickstoff unter Rühren und Eiskühlung portionsweise mit 3,0 g (43,5 mmol) Natriumnitrit versetzt. Die dunkelrote Lösung wird 12 h bei 20°C gerührt. Das Lösungsmittel wird im Vakuum abdestilliert und der Rückstand zwischen Ethylacetat (1,2 l) und gesättigter Natriumhydrogencarbonatlösung (200 ml) verteilt. Die Ethylacetatphase wird abgetrennt, mit Wasser gewaschen, getrocknet (Natriumsulfat) und eingedampft. Der Rückstand wird an Kieselgel mit Dichlormethan/Methanol 9 : 1 chromatographiert. Die Fraktion mit dem Rf 0,3 wird isoliert und mit Diisopropylether/Ethylacetat verrührt. Die gebildeten Kristalle werden abfiltriert.

Man erhält 6-(3-Dimethylaminopropyl)-1,2,3,6-tetrahydro-1,3-dioxo-4-phenyl-pyrrolo[3,4-c]carbazol in Form gelber Kristalle vom Schmp. ca. 200°C.

Das als Ausgangsprodukt verwendete 6-(3-Dimethylaminopropyl)-1,2,3,3a,4,5,6,10c-octahydro-1,3-dioxo-4-phenyl-pyrrolo[3,4-c]carbazol wird durch Umsetzung von (E)-1-[1-(3-Dimethylaminopropyl)-2-indolyl]-2-phenylethylen mit Maleinsäureimid in Toluol analog Beispiel 1, Verfahren B, hergestellt.

Das (E)-1-[1-(3-Dimethylaminopropyl)-2-indolyl)-2-phenylethylen wird wie folgt hergestellt :

11,8 g (53,8 mmol) (E)-1-(-2-indolyl)-2-phenylethylen (Herstellung siehe Beispiel 1, Verfahren B oder Can. J. Chem. 1973, 51, 792) und 8,5 g (69,9 mmol) 3-Dimethylaminopropylchlorid werden mit 9,7 g (70 mmol) Kaliumcarbonat und einer Spatelspitze Kaliumjodid in 300 ml Aceton 64 h unter Rückfluß gekocht. Das Lösungsmittel wird abdestilliert und der Rückstand zwischen Ethylacetat (300 ml) und Wasser (100 ml) verteilt. Die Ethylacetatphase wird abgetrennt, getrocknet (Natriumsulfat) und eingedampft. Der Rückstand wird an Kieselgel mit Dichlormethan/Methanol 95 : 5 getrennt. Die Fraktion mit dem Rf 0,15 wird isoliert.

Man erhält (E)-1-[1-(3-Dimethylaminopropyl)-2-indolyl]-2-phenylethylen in Form eines gelben Öls.

In analoger Weise werden die folgenden Verbindungen erhalten :

Beispiel 9.a :

4-(2-Chlorphenyl)-1,2,3,6-tetrahydro-6-(2-morpholinoethyl)-1,3-dioxo-pyrrolo[3,4-c]carbazol Schmp. 225-228°C aus Ethylacetat

Beispiel 9.b :

4-(2-Chlorphenyl)-6-(2-diethylaminoethyl)-1,2,3,6-tetrahydro-1,3-dioxo-pyrrolo[3,4-c]carbazol Schmp. 196-200°C aus Toluol

Beispiel 9.c :

4-(2-Chlorphenyl)-6-(3-dimethylaminopropyl)-1,2,3,6-tetrahydro-1,3-dioxo-pyrrolo[3,4-c]carbazol Schmp. 208-211°C aus Diisopropylether/Ethylacetat

Beispiel 9.d :

6-(3-Dimethylaminopropyl)-1,2,3,6-tetrahydro-1,3-dioxo-4-(2-trifluormethylphenyl)-pyrrolo[3,4-c]carbazol Schmp. 195-197°C aus Diisopropylether

Beispiel 9.e :

4-(2-Chlorphenyl)-6-(3-dimethylamino-2-methoxy-1-propyl)-1,2,3,6-tetrahydro-1,3-dioxo-pyrrolo [3,4-c] carbazol Schmp. 222-225°C aus Ethylacetat

Beispiel 9.f :

4-(2-Chlorphenyl)-1,2,3,6-tetrahydro-1,3-dioxo-6-(2-pyrrolidinoethyl)-pyrrolo[3,4-c]carbazol Schmp. 226-230°C aus Diisopropylether

Beispiel 9.g :

6-(3-Dimethylaminopropyl)-1,2,3,6-tetrahydro-4-(2-methylphenyl)-1,3-dioxo-pyrrolo[3,4-c]carbazol Schmp. 182-185°C aus Diisopropylether/Ethylacetat

Beispiel 9.h :

6-(3-Dimethylaminopropyl)-4-(2-fluorphenyl)-1,2,3,6-tetrahydro-1,3-dioxo-pyrrolo[3,4-c]carbazol Schmp. 202-206°C aus Diisopropylether/Ethylacetat

Beispiel 9.i :

6-(3-Diethylamino-2-hydroxy-1-propyl)-1,2,3,6-tetrahydro-1,3-dioxo-4-phenyl-pyrrolo[3,4-c]carbazol Schmp. > 190°C Zers. aus Diisopropylether

Beispiel 10 :

6-(2-Cyanoethyl)-1,2,3,6-tetrahydro-4-(2-methylphenyl)-1,3-dioxo-pyrrolo[3,4-c]carbazol

200 mg (0,52 mmol) 6-(2-Cyanoethyl)-1,2,3,3a,4,5,6,10c-octahydro-4-(2-methylphenyl)-1,3-dioxo-pyrrolo[3,4-c]carbazol und 34 mg (1,06 mmol) Schwefel in 25 ml Triethylenglycoldimethylether werden 1 h auf 220°C erhitzt. Nach dem Abkühlen werden unter Rühren 50 ml Wasser zugetropft und das ausgefallene Rohprodukt abfiltriert. Das Rohprodukt wird in Tetrahydrofuran gelöst, die Tetrahydrofuranlösung mit gesättigter Natriumchloridlösung gewaschen, getrocknet (Natriumsulfat) und eingedampft. Der Rückstand wird an Kieselgel mit Dichlormethan/Methanol 95 : 5 chromatographiert und die Fraktion mit dem Rf 0,5 isoliert.

Man erhält 6-(2-Cyanoethyl)-1,2,3,6-tetrahydro-4-(2-methylphenyl)-1,3-dioxo-pyrrolo[3,4-c]carbazol in Form gelber Kristalle, die sich ab ca. 300°C zersetzen.

Das als Ausgangsprodukt verwendete 6-(2-Cyanoethyl)-1,2,3,3a,4,5,6,10c-octahydro-4-(2-methylphenyl)-1,3-dioxo-pyrrolo[3,4-c]carbazol wird durch Umsetzung von (E)-1-[1-(2-Cyanoethyl)-2-indolyl]-2-(2-methylphenyl)ethylen mit Maleinsäureimid in Toluol analog Beispiel 1, Verfahren B, hergestellt.

Das (E)-1-[1-(2-Cyanoethyl)-2-indolyl]-2-(2-methylphenyl)ethylen wird wie folgt hergestellt :

600 mg (2,74 mmol) (E)-1-(-2-Indolyl)-2-(2-methylphenyl)ethylen (hergestellt analog Beispiel 1, Verfahren B oder Can. J. Chem. 1973, 51, 792) und 1,02 g (19,2 mmol) Acrylnitril in 30 ml Acetonitril werden mit 2 Tropfen 1,8-Diazabicyclo[5.4.o]undec-7en (DBU) versetzt und 48 h bei 20°C gerührt. Das Lösungsmittel wird abgedampft und der ölige Rückstand mit Diisopropylether/Ethylacetat (20 : 1) kristallisiert.

Man erhält (E)-1-[1-(2-Cyanoethyl)-2-indolyl]-2-(2-methylphenyl)-ethylen in Form hellgelber Kristalle.

In analoger Weise werden die folgenden Verbindungen erhalten :

Beispiel 10.a :

6-(2-Cyanoethyl)-1,2,3,6-tetrahydro-1,3-dioxo-4-phenyl-pyrrolo[3,4-c]carbazol Schmp. Zers. ab 300°C aus Diisopropylether

Beispiel 10.b :

6-(2-Cyanoethyl)-4-(2-fluorphenyl)-1,2,3,6-tetrahydro-1,3-dioxo-pyrrolo[3,4-c]carbazol Schmp. Zers. ab 300°C aus Dichlormethan/Methanol

## Ansprüche

1. Pyrrolocarbazol-Derivate der allgemeinen Formel 1

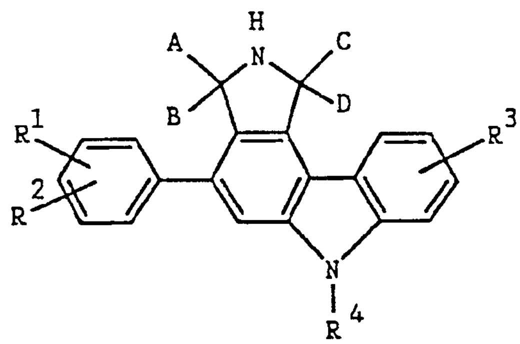

(I)

in welcher A und B oder C und D entweder gleich sind und Wasserstoff bedeuten oder zusammen ein Carbonylsauerstoffatom bilden, oder verschieden sind, wobei einer der Reste von A und B oder von C und D für Wasserstoff steht und der andere von den beiden Resten eine Hydroxygruppe bedeutet, mit der Maßgabe, daß mindestens eine der Kombinationen A und B oder C und D zusammen ein Carbonylsauerstoffatom bilden, $R^1$, $R^2$ und $R^3$ entweder gleich oder verschieden sind und jeweils für Wasserstoff, Halogen, Trifluormethyl, eine Alkylgruppe mit 1 bis 4 C-Atomen, eine Nitrogruppe, eine Aminogruppe, unsubstituiert oder substituiert durch eine Alkylgruppe mit 1 bis 4 C-Atomen oder eine Benzylgruppe, eine Alkoxygruppe mit 1 bis 4 C-Atomen, eine Hydroxygruppe, eine Acylgruppe mit 1 bis 4 C-Atomen oder eine Aminoalkoxygruppe mit 1 bis 12 C-Atomen, unsubstituiert oder substituiert an der Alkylkette durch Alkylreste mit 1 bis 4 C-Atomen, Hydroxy oder Alkoxy mit 1 bis 4 C-Atomen und/oder substituiert am Stickstoffatom durch Alkylreste mit 1 bis 4 C-Atomen oder Benzyl oder bei der zwei Substituenten am Stickstoffatom oder ein Substituent am Stickstoffatom mit einem Substituenten der Alkylkette und zusammen mit dem Stickstoffatom einen heterozyklischen Ring mit 3 bis 6 C-Atomen bilden, der auch Sauerstoff-, Schwefel- und/oder weitere Stickstoffatome enthalten und durch Alkylreste mit 1 bis 4 C-Atomen substituiert sein kann, stehen und $R^4$ für Wasserstoff, eine geradkettige oder verzweigte Alkylgruppe mit 1 bis 6 C-Atomen, eine Cyanoalkylgruppe mit 2 bis 4 C-Atomen, einen Alkoxycarbonylalkylrest mit bis zu 7 C-Atomen oder eine Aminoalkylgruppe mit 1 bis 12 C-Atomen, unsubstituiert oder substituiert an der Alkylkette durch Alkylreste mit 1 bis 4 C-Atomen, Hydroxy oder Alkoxy mit 1 bis 4 C-Atomen und/oder substituiert am Stickstoffatom durch Alkylreste mit 1 bis 4 C-Atomen oder Benzyl oder bei der zwei Substituenten am Stickstoffatom oder ein Substituent am Stickstoffatom mit einem Substituenten der Alkylkette und zusammen mit dem Stickstoffatom einen heterozyklischen Ring mit 3 bis 6 C-Atomen bilden, der auch Sauerstoff-, Schwefel- und/oder weitere Stickstoffatome enthalten und durch Alkylreste mit 1 bis 4 C-Atomen substituiert sein kann, steht sowie deren pharmakologisch unbedenkliche Salze.

2. Verbindungen der allgemeinen Formel I gemäß Anspruch 1, in denen $R^1$, $R^2$ und $R^3$ entweder gleich oder verschieden sind und Wasserstoff, Fluor, Chlor, Brom, eine Trifluormethyl-, Hydroxy-, Nitro-, Methyl, Ethyl-, n-Propyl-, Isopropyl-, n-Butyl-, Amino-, Methoxy-, Ethoxy-, Aminoethoxy-, Aminopropoxy-, Dimethylaminoethoxy-, Dimethylaminopropoxy-, Diethylaminoethoxy-, N-Benzyl-N-methylaminoethoxy-, N-Benzyl-N-methylaminopropoxy-, Dimethylaminohydroxypropoxy-, Piperidinoethoxy-, Piperidinopropoxy-, Pyrrolidinoethoxy-, Pyrrolidinopropoxy-, Morpholinoethoxy-, Morpholinopropoxy-, Pyrrolidinylmethoxy-, Piperidinylmethoxy-, N-Methylpiperidinylmethoxy-, N-Methyl-pyrrolidinylmethoxy-, Dimethylamino-methoxypropoxy-, Formyl, Acetyl, Propionyloder eine Butyrylgruppe bedeuten und $R^4$ Wasserstoff, eine Methyl-, Ethyl-, n-Propyl-, Isopropyl-, n-Butyl-, Cyanomethyl-, Cyanoethyl-, Methoxycarbonylethyl-, Ethoxycarbonylethyl-,

Methoxycarbonylmethyl-, Aminoethyl-, Aminopropyl-, Dimethylaminoethyl-, Dimethylaminopropyl-, Diethylaminoethyl-, N-Benzyl-N-methylaminoethyl-, N-Benzyl- N-methylaminopropyl-, Dimethylaminohydroxypropyl-, Diethylaminohydroxypropyl-, Piperidinoethyl-, Piperidinopropyl-, Pyrrolidinoethyl-, Pyrrolidinopropyl-, Morpholinoethyl-, Morpholinopropyl-, Pyrrolidinylmethyl-, Piperidinylmethyl-, N-Methylpiperidinylmethyl-, Dimethylaminomethoxypropyl-, Diethylaminomethoxypropyl- oder N-Methyl-pyrrolidinylmethylgruppe bedeutet.

3. Verfahren zur Herstellung von Verbindungen der allgemeinen Formel I gemäß der Ansprüche 1 und 2 dadurch gekennzeichnet, daß entweder

a) für den Fall, daß sowohl A und B als auch C und D ein Carbonylsauerstoffatom bilden, $R^1$, $R^2$ und $R^3$ die oben genannte Bedeutung haben und $R^4$ Wasserstoff bedeutet, 1-Aryl-4-(2-nitroaryl)-1,4-butadiene der allgemeinen Formel II

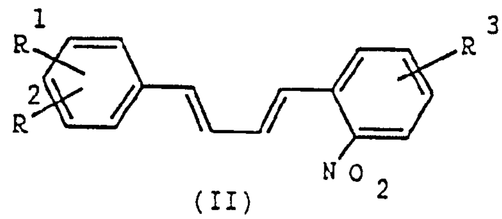

(II)

in der $R^1$, $R^2$ und $R^3$ die oben genannte Bedeutung haben, durch Cycloaddition mit Maleinimid zu Verbindungen der allgemeinen Formel III

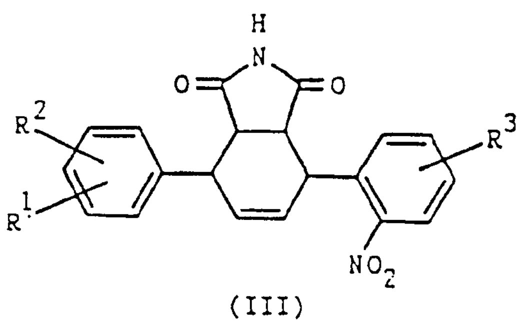

(III)

umgesetzt werden, diese zu Verbindungen der allgemeinen Formel IV

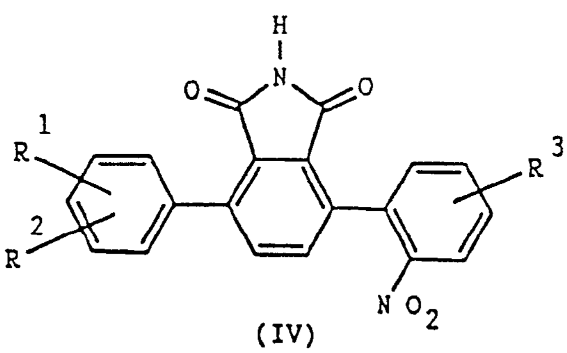

(IV)

dehydriert und diese durch Erhitzen mit Phosphor(III)- Verbindungen in einem inerten Lösungsmittel zu Pyrrolocarbazol-Derivate der allgemeinen Formel Ia

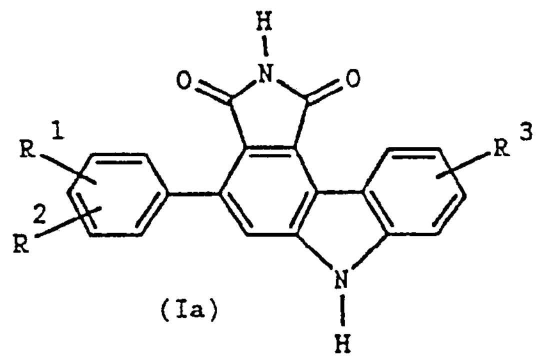

(Ia)

umgesetzt werden,

b) für den Fall, daß sowohl A und B als auch C und D ein Carbonylsauerstoffatom bilden und $R^1$, $R^2$, $R^3$ und $R^4$ die oben angegebene Bedeutung haben, unsubstituierte oder substituierte Indol-2-aldehyde oder am Indolstickstoffatom substituierte Derivate der allgemeinen Formel V

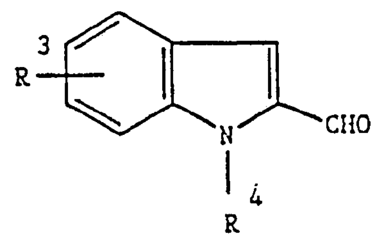

(V)

in der $R^3$ und $R^4$ die oben angegebene Bedeutung haben, durch eine Wittig-Reaktion mit geeignet substituierten Arylmethyl-triphenylphosphoniumhalogeniden der allgemeinen Formel VI,

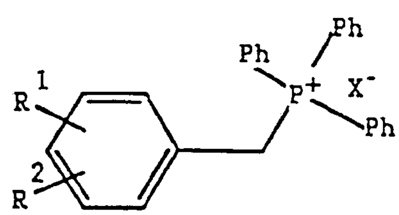

(VI)

in der $R^1$ und $R^2$ die oben angegebene Bedeutung haben und X Halogen, vorzugsweise Chlor oder Brom bedeutet, in Gegenwart geeigneter Basen, wie z.B. Alkalicarbonaten, -alkoholaten, -amiden oder lithiumorganischen Verbindungen, zu 2-(2-Arylvinyl)indolen der allgemeinen Formel VII

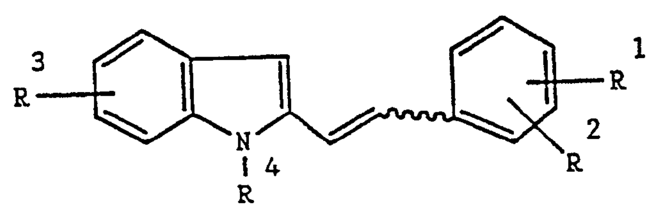

(VII)

umgesetzt werden, die je nach Reaktionsbedingungen als cis-/trans-Isomerengemische erhalten werden, die durch übliche Verfahren wie Kristallisation oder Chromatographie trennbar sind und die als Isomer oder

Isomerengemisch durch Erhitzen mit Maleinimid in einem geeigneten Lösungsmittel in die Verbindungen der allgemeinen Formel VIII

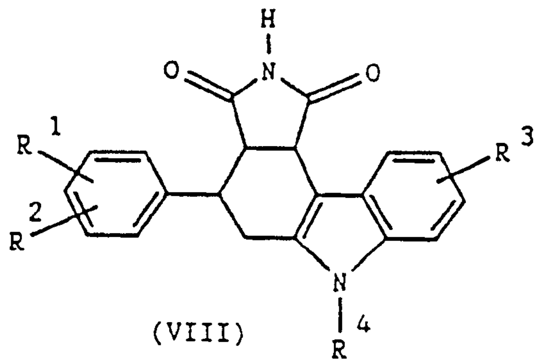

(VIII)

umgesetzt werden und zu Verbindungen der allgemeinen Formel Ib

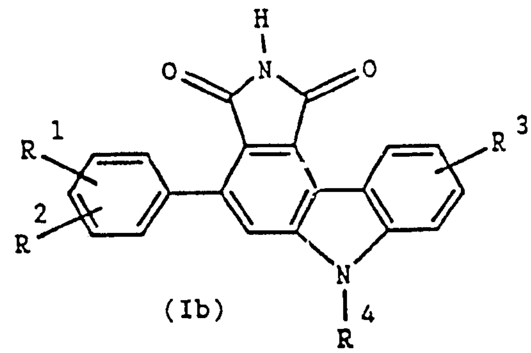

(Ib)

dehydriert werden und

c) Verbindungen der allgemeinen Formeln Ia oder Ib gewünschtenfalls durch Reduktion in Verbindungen der allgemeinen Formel I übergeführt werden, bei denen entweder A und B oder C und D zusammen ein Carbonylsauerstoffatom bilden und die anderen beiden Reste entweder beide Wasserstoff sind oder einer der beiden anderen Reste für Wasserstoff und der andere für eine Hydroxygruppe steht und die teilweise anfallenden Regioisomerengemische durch übliche Verfahren der Kristallisation oder Chromatographie getrennt werden und

d) gewünschtenfalls Verbindungen der allgemeinen Formel Ic, bei denen $R^4$ für Wasserstoff steht, durch Umsetzung mit Verbindungen $R^{4'}$-X, bei denen $R^{4'}$ mit Ausnahme von Wasserstoff die für $R^4$ angegebenen Bedeutungen besitzt und X Halogen bedeutet, in Gegenwart von Basen wie Hydriden, Carbonaten, Hydroxiden, Oxiden oder Alkoxiden der Alkali- oder Erdalkalimetalle, oder von lithiumorganischen Verbindungen in an sich bekannter Weise am Indolstickstoffatom zu Lactamen der allgemeinen Formel I alkyliert werden, bei denen $R^4$ die oben genannten Bedeutungen mit Ausnahme von Wasserstoff hat, oder

e) gewünschtenfalls Verbindungen der allgemeinen Formel I, bei denen $R^1$ und/oder $R^2$ und/oder $R^3$ für eine Alkoxygruppe mit 1 bis 4 C-Atomen stehen, in an sich bekannter Weise durch Etherspaltung in Verbindungen der allgemeinen Formel I, bei denen $R^1$ und/oder $R^2$ und/oder $R^3$ für eine Hydroxygruppe stehen, umgesetzt werden oder

f) gewünschtenfalls Verbindungen der allgemeinen Formel I, bei denen $R^1$ und/oder $R^2$ und/oder $R^3$ eine Nitrogruppe bedeuten, in an sich bekannter Weise durch Reduktion in Verbindungen der allgemeinen Formel I, bei denen $R^1$ und/oder $R^2$ und/oder $R^3$ für eine unsubstituierte Aminogruppe stehen, übergeführt werden oder

g) gewünschtenfalls Verbindungen der allgemeinen Formel I, bei denen $R^1$ und/oder $R^2$ und/oder $R^3$ für eine Hydroxygruppe stehen, in an sich bekannter Weise durch Aminoalkylierung in Verbindungen der allgemeinen Formel I, bei denen $R^1$ und/oder $R^2$ und/oder $R^3$ für eine unsubstituierte oder substituierte Aminoalkoxygruppe stehen, umgesetzt werden.

4. Arzneimittel enthaltend neben üblichen Hilfs- und Zusatzstoffen Verbindungen der allgemeinen Formel I gemäß Anspruch 1.

5. Verwendung von Verbindungen der allgemeinen Formel I gemäß Anspruch 1 zur Herstellung von Pharmazeutika zur Prävention und/oder Behandlung von Herz- und Gefäßkrankheiten wie Thrombosen, Arteriosklerose, Hypertonien, von Entzündungsprozessen, Allergien, Krebs und degenerativen Schäden des Zentralnervensystems und zur Behandlung viraler Erkrankungen.

**Claims**

1. Pyrrolocarbazole derivatives of the general formula I

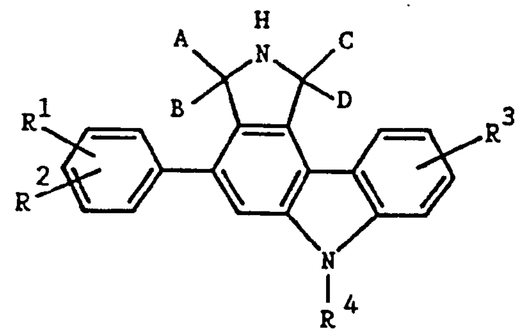

(I)

in which A and B or C and D are either the same and signify hydrogen or together form a carbonyl oxygen atom or are different, whereby one of the radicals of A and B or of C and D stands for hydrogen and the other of the two radicals signifies a hydroxyl group, with the proviso that at least one of the combinations A and B or C and D together form a carbonyl oxygen atom, $R^1$, $R^2$ and $R^3$ are either the same or different and,in each case,stand for hydrogen, halogen, trifluoromethyl, an alkyl group with 1 to 4 C-atoms, a nitro group, an amino group, unsubstituted or substituted by an alkyl group with 1 to 4 C-atoms or a benzyl group, an alkoxy group with 1 to 4 C-atoms, a hydroxyl group, an acyl group with 1 to 4 C-atoms or an aminoalkoxy group with 1 to 12 C-atoms, unsubstituted or substituted on the alkyl chain by alkyl radicals with 1 to 4 C-atoms, hydroxyl or alkoxy with 1 to 4 C-atoms and/or substituted on the nitrogen atom by alkyl radicals with 1 to 4 C-atoms or benzyl or in which two substituents on the nitrogen atom or one substituent on the nitrogen atom with a substituent of the alkyl chain together with the nitrogen atom form a heterocyclic ring with 3 to 6 C-atoms, which can also contain oxygen, sulphur and/or further nitrogen atoms and can be substituted by alkyl radicals with 1 to 4 C-atoms,and $R^4$ stands for hydrogen, a straight-chained or branched alkyl group with 1 to 6 C-atoms, a cyanoalkylgroup with 2 to 4 C-atoms, an alkoxycarbonylalkyl radical with up to 7 C-atoms or an aminoalkyl group with 1 to 12 C-atoms, unsubstituted or substituted on the alkyl chain by alkyl radicals with 1 to 4 C-atoms, hydroxyl or alkoxy with 1 to 4 C-atoms and/or substituted on the nitrogen atom by alkyl radicals with 1 to 4 C-atoms or benzyl or in which two substituents on the nitrogen atom or one substituent on the nitrogen atom with a substituent of the alkyl chain together with the nitrogen atom form a heterocyclic ring with 3 to 6 C-atoms, which can also contain oxygen, sulphur and/or further nitrogen atoms and can be substituted by alkyl radicals with 1 to 4 C-atoms, as well as their pharmacologically acceptable salts.

2. Compounds of the general formula I according to claim 1, in which $R^1$, $R^2$ and $R^3$ are either the same or different and signify hydrogen, fluorine, chlorine, bromine, a trifluoromethyl, hydroxyl, nitro, methyl, ethyl, n-propyl, isopropyl, n-butyl, amino, methoxy, ethoxy, aminoethoxy, aminopropoxy, dimethylaminoethoxy, dimethylaminopropoxy, diethylaminoethoxy, N-benzyl-N-methylaminoethoxy, N-benzyl- N-methylaminopropoxy, dimethylaminohydroxypropoxy, piperidinoethoxy, piperidinopropoxy, pyrrolidinoethoxy, pyrrolidinopropoxy, morpholinoethoxy, morpholinopropoxy, pyrrolidinylmethoxy, piperidinylmethoxy, N-methylpiperidinylmethoxy, N-methylpyrrolidinylmethoxy, dimethylaminomethoxypropoxy, formyl, acetyl, propionyl or a butyryl group and $R^4$ signifies hydrogen, a methyl, ethyl, n-propyl, isopropyl, n-butyl, cyanomethyl, cyanoethyl, methoxycarbonylethyl, ethoxycarbonylethyl, methoxycarbonylmethyl, aminoethyl, aminopropyl, dimethylaminoethyl, dimethylaminopropyl, diethylaminoethyl, N-benzyl-N-methylaminoethyl, N-benzyl-N-methylaminopropyl, dimethylaminohydroxypropyl, diethylaminohydroxypropyl, piperidinoethyl, piperidinopropyl, pyrrolidinoethyl, pyrrolidinopropyl, morpholinoethyl, morpholinopropyl, pyrrolidinylmethyl, piperidinylmethyl, N-methyl-piperidinylmethyl, dimethylaminomethoxypropyl, diethylaminomethoxypropyl or N-methylpyrrolidinylmethyl group.

3. Process for the preparation of compounds of the general formula I according to claims 1 and 2, characterised in that either

a) for the case that not only A and B but also C and D form a carbonyl oxygen atom, $R^1$, $R^2$ and $R^3$ have the above-mentioned meaning and $R^4$ signifies hydrogen, 1-aryl-4-(2-nitroaryl)-1,4-butadienes of the general formula II

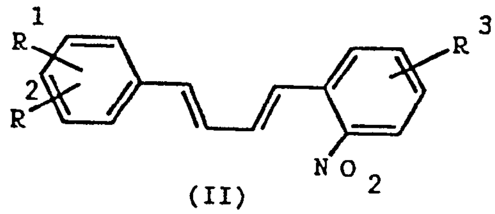

(II)

in which $R^1$, $R^2$ and $R^3$ have the above-mentioned meaning, are reacted by cycloaddition with maleinimide to compounds of the general formula III

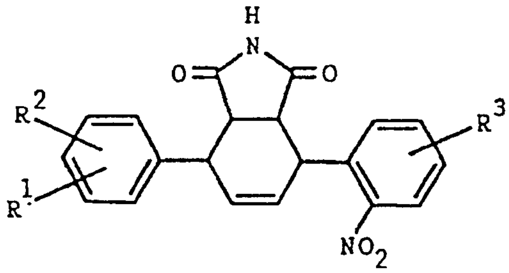

(III)

these dehydrogenated to compounds of the general formula IV

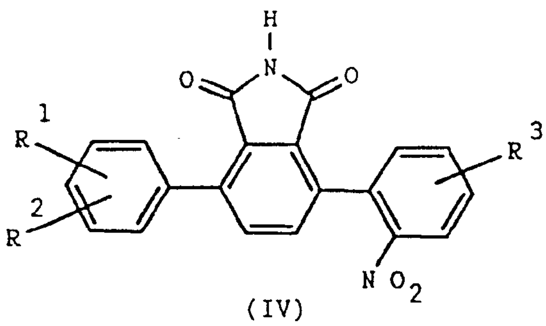

(IV)

and these reacted by heating with phosphorus (III) compounds in an inert solvent to pyrrolocarbazole derivatives of the general formula Ia

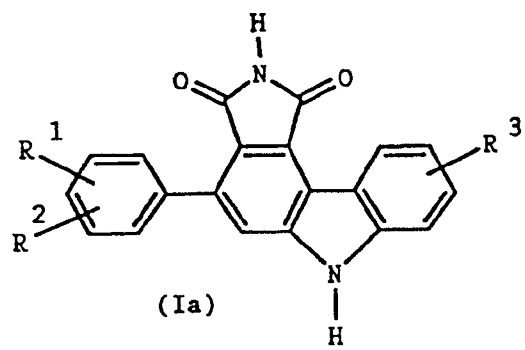

(Ia)

b) for the case that not only A and B but also C and D form a carbonyl oxygen atom and $R^1$, $R^2$, $R^3$ and $R^4$

have the above-given meaning, unsubstituted or substituted indole-2-aldehydes or derivatives substituted on the indole nitrogen atom of the general formula V

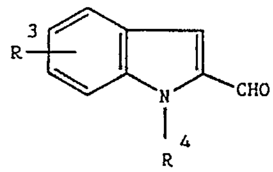

(V)

in which $R^3$ and $R^4$ have the above-given meaning, are reacted by a Wittig reaction with suitably substituted arylmethyl-triphenylphosphonium halides of the general formula VI

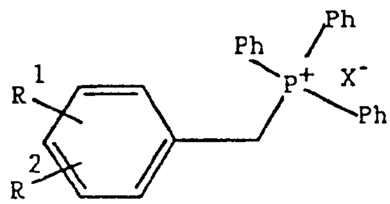

(VI)

in which $R^1$ and $R^2$ have the above-given meaning and X signifies halogen, preferably chlorine or bromine, in the presence of suitable bases, such as e.g. alkali carbonates, alcoholates, amides or lithium organic compounds, to 2-(2-arylvinyl)-indoles of the general formula VII

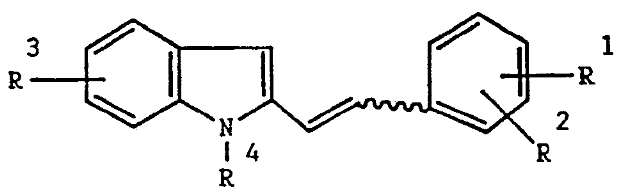

(VII)

which, depending upon the reaction conditions, are obtained as cis/trans-isomer mixtures which are separable by usual processes, such as crystallisation or chromatography, and which are reacted as isomer or isomer mixture by heating with maleinimide in a suitable solvent into the compounds of the general formula VIII

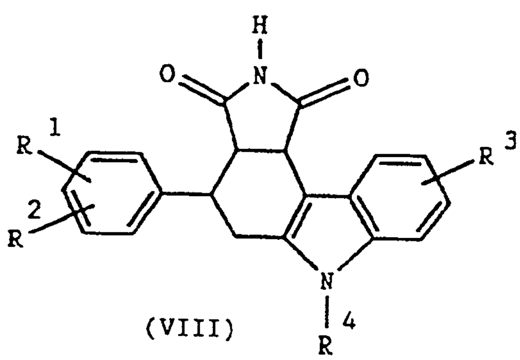

(VIII)

and are dehydrogenated to compounds of the general formula Ib

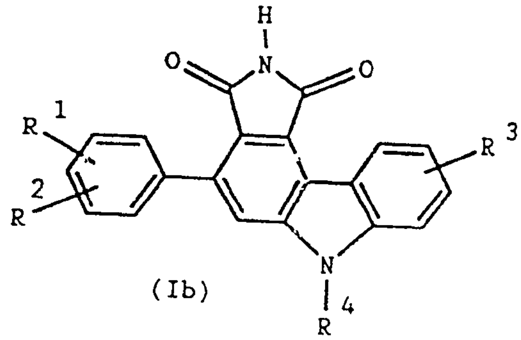

(Ib)

and

c) compounds of the general formulae Ia or Ib are, if desired, converted by reduction into compounds of the general formula I in which either A and B or C and D together form a carbonyl oxygen atom and the two other radicals are either both hydrogen or one of the two other radicals stands for hydrogen and the other for a hydroxyl group and the partly obtained regioisomeric mixtures are separated by usual processes of crystallisation or chromatography and

d) if desired, compounds of the general formula Ic, in which $R^4$ stands for hydrogen, are alkylated by reaction with componds $R^{4'}$-X, in which $R^{4'}$ possesses, with the exception of hydrogen, the meanings given for $R^4$ and X signifies halogen, in the presence of bases, such as hydrides, carbonates, hydroxides, oxides or alkoxides of the alkali or alkaline earth metals, or of organo-lithium compounds in per se known manner on the indole nitrogen atom to lactams of the general formula I, in which $R^4$ has the above-mentioned meanings with the exception of hydrogen, or

e) if desired, compounds of the general formula I, in which $R^1$ and/or $R^2$ and/or $R^3$ stand for an alkoxy group with 1 to 4 C-atoms, are reacted in per se known way by ether cleavage into compounds of the general formula I, in which $R^1$ and/or $R^2$ and/or $R^3$ stand for a hydroxyl group, or

f) if desired, compounds of the general formula I, in which $R^1$ and/or $R^2$ and/or $R^3$ signify a nitro group, are converted in per se known manner by reduction into compounds of the general formula I, in which $R^1$ and/or $R^2$ and/or $R^3$ stand for an unsubstituted amino group, or

g) if desired, compounds of the general formula I, in which $R^1$ and/or $R^2$ and/or $R^3$ stand for a hydroxyl group, are reacted in per se known way by aminoalkylation into compounds of the general formula I, in which $R^1$ and/or $R^2$ and/or $R^3$ stand for an unsubstituted or substituted aminoalkoxy group.

4. Medicaments containing, besides usual adjuvant and additive materials, compounds of the general formula I according to claim 1.

5. Use of compounds of the general formula I according to claim 1 for the preparation of pharmaceuticals for the prevention and/or treatment of heart and vessel diseases, such as thromboses, arterioscleroses, hypertonia, of inflammatory processes, allergies, cancers and degenerative damage of the central nervous system and for the treatment of viral diseases.

## Revendications

1. Dérivés de pyrrolocarbazole répondant à la formule générale I :

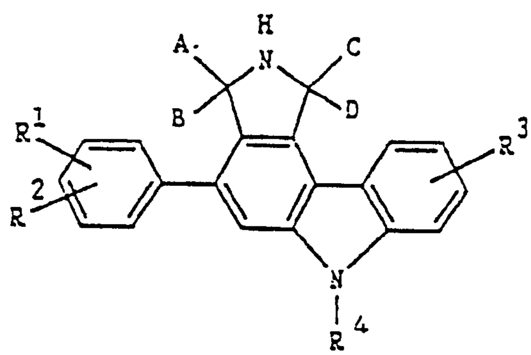

(I)

dans laquelle :

A et B ou C et D soit sont identiques et consistent en un atome d'hydrogène, ou forment ensemble l'atome d'oxygène d'un radical carbonyle, soit sont différents, l'un des restes A et B ou l'un des restes C et D représentant alors un atome d'hydrogène et l'autre un groupe hydroxy, sous réserve qu'au moins l'une des combinaisons A et B ou C et D forme l'atome d'oxygène d'un radical carbonyle ;

$R^1$, $R^2$ et $R^3$ identiques ou différents, représentent un atome d'hydrogène, un atome d'halogène,. un radical trifluorométhyle, un groupe alkyle renfermant de 1 à 4 atomes de carbone, un groupe nitro, un groupe amino, non substitué ou substitué par un groupe alkyle renfermant de 1 à 4 atomes de carbone ou un groupe benzyle, un groupe alkoxy renfermant de 1 à 4 atomes de carbone, un groupe hydroxy, un groupe acyle renfermant de 1 à 4 atomes de carbone ou un groupe aminoalkoxy renfermant de 1 à 12 atomes de carbone, non substitué ou substitué sur-la chaîne alkyle par des radicaux alkyles renfermant de 1 à 4 atomes de carbone, hydroxy ou alkoxy renfermant de 1 à 4 atomes de carbone et/ou substitué sur l'atome d'azote par des radicaux alkyles renfermant 1 à 4 atomes de carbone ou benzyles ou forment sur les deux substituants de l'atome d'azote ou sur un substituant de l'atome d'azote avec un substituant de la chaîne alkyle et avec l'atome d'azote, un cycle hétérocyclique renfermant de 3 à 6 atomes de carbone contenant encore des atomes d'oxygène, de soufre et/ou d'autres atomes d'azote, et peut être substitué par des radicaux alkyles renfermant de 1 à 4 atomes de carbone ; et

$R^4$ représente un atome d'hydrogène, un groupe alkyle à chaîne droite ou ramifiée renfermant de 1 à 6 atomes de carbone, un groupe cyanoalkyle renfermant de 2 à 4 atomes de carbone, un reste alkoxycarbonylalkyle renfermant jusqu'à 7 atomes de carbone ou un groupe aminoalkyle renfermant de 1 à 12 atomes de carbone non-substitué ou substitué sur la chaîne alkyle par des radicaux alkyles renfermant de 1 à 4 atomes de carbone, hydroxy ou alkoxy renfermant de 1 à 4 atomes de carbone, et/ou substitué sur l'atome d'azote du groupe amino par des radicaux alkyles renfermant de 1 à 4 atomes de carbone, ou benzyles ou forment sur les deux substituants de l'atome d'azote ou sur un substituant de l'atome d'azote avec un substituant de la chaîne alkyle et avec l'atome d'azote, un cycle hétérocyclique renfermant de 3 à 6 atomes de carbone qui contient aussi des atomes d'oxygène, de soufre, et/ou d'autres atomes d'azote et qui peut être substitué par des radicaux alkyles renfermant de 1 à 4 atomes de carbone, ainsi que leurs sels pharmacologiquement acceptables.

2. Dérivés répondant à la formule générale I d'après la revendication 1, dans lesquels $R^1$, $R^2$ et $R^3$ sont soit identiques, soit différents, et représentent un atome d'oxygène, de fluor, de chlore, de brome, un radical trifluorométhyle, un groupe hydroxy, nitro, un radical méthyle, éthyle, n-propyle, isopropyle, n-butyle, un groupe amino, méthoxy, éthoxy, aminoéthoxy, aminopropoxy, diméthylaminoéthoxy, diméthylaminopropoxy, diéthylaminoéthoxy, N-benzyl- N-méthylaminoéthoxy, N-benzyl-N-méthylaminopropoxy, diméthylaminohydroxypropoxy, pipéridinoéthoxy, pipéridinopropoxy, pyrrolidinoéthoxy, pyrrolidinopropoxy, morpholinoéthoxy, morpholinopropoxy, pyrrolidinylméthoxy, pipéridinylméthoxy, N-méthylpipéridinylméthoxy, N-méthyl-pyrrolidinylméthoxy, diméthylaminométhoxypropoxy, un radical formyle, acétyle, propionyle ou un groupe butyryle et $R^4$ représente un atome d'hydrogène, un'radical méthyle, éthyle, n-propyle, isopropyle, n-butyle, aminoéthyle, aminopropyle, diméthylaminoéthyle, diméthylaminopropyle, diéthylaminoéthyle, N-benzyl-N-méthylaminoéthyle, N-benzyl-N-méthylaminopropyle, diméthylaminohydroxypropyle, diéthylaminohydroxypropyle, pipéridinoéthyle, pipéridinopropyle, pyrrolidinoéthyle, pyrrolidinopropyle, morpholinoéthyle, morpholinopropyle, pyrrolidinylméthyle, pipéridinylméthyle, N-méthylpipéridinylméthyle, diméthylaminométhoxypropyle, diéthylaminométhoxypropyle, N-méthyl-pyrrolidinylméthyle.

3. Procédé de préparation de dérivés répondant à la formule générale I selon les revendications 1 et 2, caractérisé en ce que dans le cas où

a) aussi bien A et B que C et D forment l'atome d'oxygène d'un radical carbonyle, $R^1$, $R^2$ et $R^3$ ont la signification indiquée ci-dessus et $R^4$ représente un atome d'hydrogène, on fait réagir du 1-aryl-4-(2-nitroaryl)1,4-butadiène de formule générale II

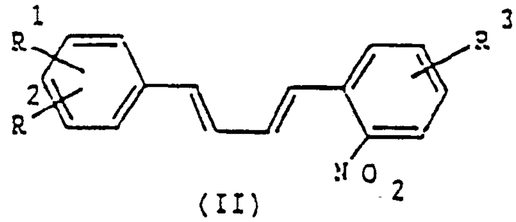

(II)

dans laquelle $R^1$, $R^2$ et $R^3$ présentent les significations indiquées ci-dessus, par cycloaddition avec la maléinimide pour obtenir des dérivés de formule générale III

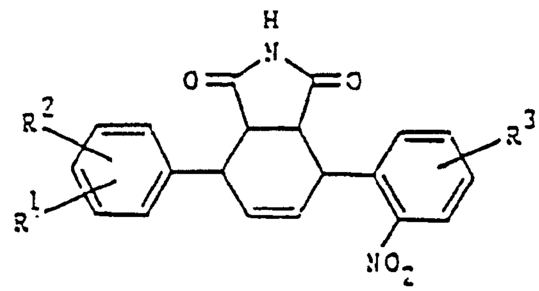

(III)

que l'on déshydrogène pour obtenir des dérivés de formule générale IV

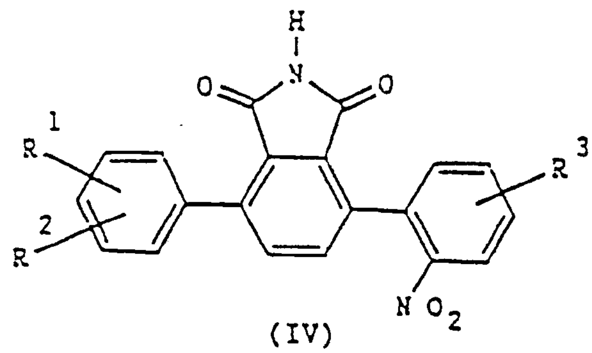

(IV)

et ces derniers sont traités par chauffage de dérivés de phosphore (III), dans un solvant inerte, pour obtenir des dérivés de pyrrolocarbazole répondant à la formule générale Ia

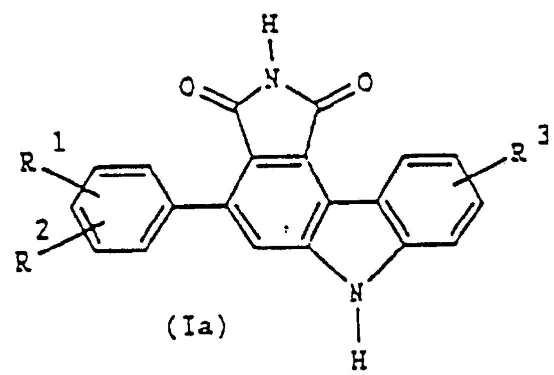

(Ia)

b) aussi bien A et B que C et D forment l'atome d'oxygène d'un radical carbonyle et $R^1$, $R^2$, $R^3$ et $R^4$ ont les significations indiquées ci-dessus, on fait réagir des indol-2-aldéhydes non substituées ou substituées ou des dérivés de l'indole substitués sur l'atome d'azote de formule générale V

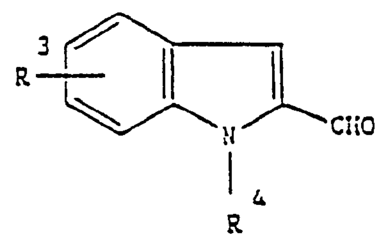

(V)

dans laquelle $R^3$ et $R^4$ ont la signification indiquée ci-dessus, par une réaction de Wittig avec des halogénures d'arylméthyltriphénylphosphonium substitués de façon appropriée, de formule générale VI

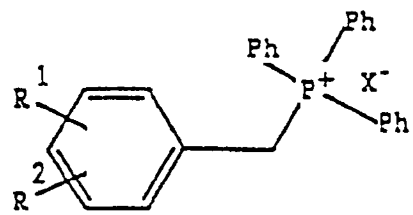

(VI)

dans laquelle $R^1$ et $R^2$ ont la signification indiquée ci-dessus et X représente un halogène, de préférence un atome de chlore ou de brome, en présence de bases appropriées telles que des carbonates, alcoolates, des amides alcalines ou des dérivés organiques du lithium pour obtenir des 2-(2-arylvinyl)indoles de formule générale VII

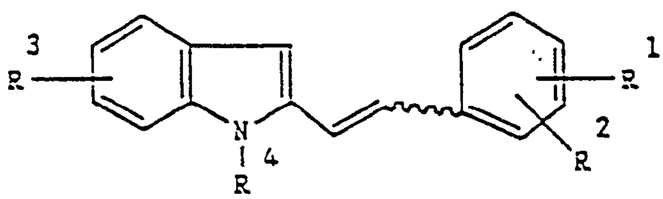

(VII)

qui, selon les conditions de la réaction, sont obtenus sous la forme de mélanges d'isomères cis/trans que l'on peut séparer par des procédés habituels tels que cristallisation ou chromatographie et que l'on fait réagir sous la forme d'isomères ou de mélanges d'isomères, par chauffage en présence de maléinimide dans un solvant approprié pour obtenir des dérivés de formule générale VIII

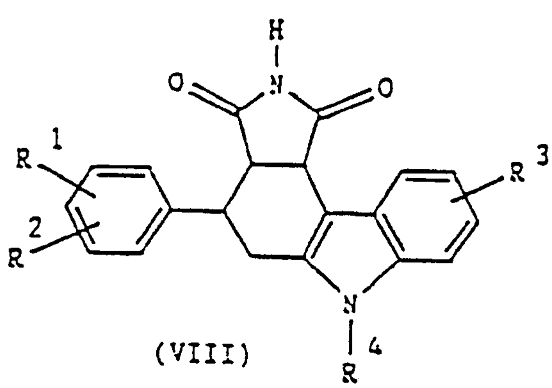

(VIII)

et que l'on déshydrogène pour obtenir les dérivés de formule générale Ib

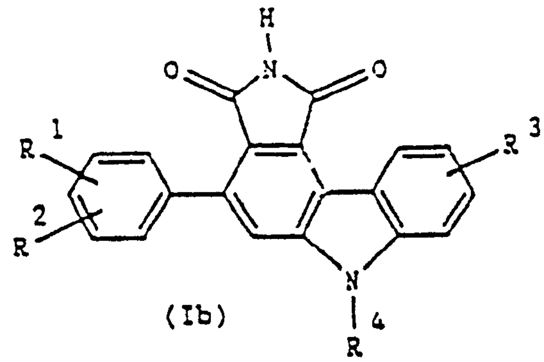

(Ib)

et

c) les dérivés de formule générale Ia ou Ib sont éventuellement transformés par réduction en dérivés de formule générale I, dans lesquels soit A et B soit C et D forment l'atome d'oxygène d'un radical carbonyle et les deux autres restes représentent deux atomes d'hydrogène ou l'un des deux autres restes représente un atome d'hydrogène et l'autre représente un groupe hydroxy, et les mélanges d'isomères géographiques partiellement produits sont séparés par des procédés usuels tels que cristallisation ou chromatographie et

d) le cas échéant, les dérivés de formule générale Ic dans lesquels $R^4$ représente un atome d'hydrogène, par réaction avec des dérivés $R^{4'}$-X dans lesquels $R^{4'}$ présente les significations indiquées pour $R^4$, à l'exception de l'hydrogène, et X représente un atome d'halogène, en présence de bases telles que des hydrures, carbonates, hydroxydes, oxydes ou alkoxydes de métaux alcalins ou alcalino-terreux ou des dérivés organiques du lithium, sont, d'une façon connue en soi, alkylés sur de l'indole en lactames de formule générale I, dans lesquels $R^4$ présente les significations indiquées ci-dessus à l'exception de l'hydrogène, ou bien

e) le cas échéant, les dérivés de formule générale I dans lesquels $R^1$ et/ou $R^2$ et/ou $R^3$ représentent un groupe alkoxy renfermant de 1 à 4 atomes de carbone sont transformés, de façon connue en soi, par scission de l'éther en dérivés de formule générale I dans lesquels $R^1$ et/ou $R^2$ et/ou $R^3$ représentent un groupe hydroxy, ou bien

f) le cas échéant, des dérivés de formule générale I dans lesquels $R^1$ et/ou $R^2$ et/ou $R^3$ représentent un groupe nitro sont transformés d'une façon connue en soi par réduction en dérivés de formule générale I dans lesquels $R^1$ et/ou $R^2$ et/ou $R^3$ représentent un groupe amino non substitué, ou bien.

g) le cas échéant, des dérivés de formule générale I dans lesquels $R^1$ et/ou $R^2$ et/ou $R^3$ représentent un groupe hydroxy sont transformés de façon connue en soi par aminoalkylation en dérivés de formule générale I dans lesquels $R^1$ et/ou $R^2$ et/ou $R^3$ représentent un groupe aminoalkoxy non substitué ou substitué.

4. Médicament contenant, en plus des auxiliaires ou additifs usuels, des dérivés de formule générale I selon la revendication 1.

5. Utilisation de dérivés de formule générale I selon la revendication 1 pour la préparation de produits pharmaceutiques pour la prévention et/ou le traitement de maladies cardiaques ou vasculaires telles que thrombose, artériosclérose, hypertension, de processus inflammatoires, d'allergies, de cancers et de dommages dégénératifs du système nerveux central et pour le traitement de maladies virales.